# EUROPEAN PATENT APPLICATION

(11) **EP 4 162 948 A1**
(43) Date of publication of application: **12.04.2023**
(21) Application number: 21306398.5
(22) Date of filing: 05.10.2021
(51) Int. Cl.: A61K 39/00, A61K 39/012, A61K 39/085, A61K 39/12, A61P 3/10, A61P 31/04, A61P 31/14, A61P 33/02, A61P 37/08

(54) **TRANSCRIPTION INFIDELITY DERIVED CHIMERIC IMMUNOGENS AND USES THEREOF**

(71) Applicant: Genclis, 54500 Vandoeuvre-les-Nancy (FR)
(72) Inventor: BIHAIN, Bernard, 54000 NANCY (FR); THOUVENOT, Benoît, 54230 CHALIGNY (FR); ROITEL, Olivier, 54210 SAINT NICOLAS DE PORT (FR); TOMASINA, Julie, 54110 VARANGEVILLE (FR); JACQUENET, Sandrine, 54600 VILLERS-LES-NANCY (FR)
(74) Representative: Cabinet Becker et Associés

(57) **Abstract**

This application invention relates to compositions and methods for inducing or stimulating an immune response in a subject. The invention more particularly relates to transcription infidelity derived chimeric immunogens and their uses. The invention may be used to produce strong and calibrated humoral response against any antigen, such as self and non-self antigens, in any organism.

## Description

This invention relates to compositions and methods for inducing or stimulating an immune response in a subject. The invention more particularly relates to Transcription Infidelity derived chimeric immunogens and their uses. The invention may be used to produce strong and calibrated humoral response against any antigen, such as self- and non-self antigens, in any organism.

### Background

The inventors have previously described a mechanism that causes single base transcriptional frameshifts at the RNA level. Such mechanism, termed Transcription Infidelity ("TI"), was disclosed for instance in WO2008/009751. The inventors have further discovered that TI is involved in the molecular origin of allergy (see WO2017/158202, Thouvenot, 2020).

Continuing their research, the inventors have now further discovered that TI defines natural humoral immunity, and can be harnessed by creating non-natural chimeric immunogens (as described below) that elicit production of specific high affinity antibodies to any selected antigen of interest.

The present application therefore discloses a single, unique and previously unsuspected mechanism that is central to normal education of the immune system involved in numerous and diversified pathological situations. This application also discloses, for the first time, a method of TI-assisted sequential immunization (TIASI) *in vivo.*

Applied to the Covid-19 agent, the present application discloses that TIASI allows the production of anti-SARS-CoV2 neutralizing antibodies in immunized mice.

### Summary of the Invention

In one aspect, the invention provides a chimeric immunogen comprising (i) one or more antigen(s) or fragment(s) thereof, covalently coupled to (ii) one or more TI segments. The coupling between antigen(s) and TI segment(s) is preferably covalent, through a peptide bond, direct or through a linker group, either at C-terminal extremity or N-terminal extremity, or at both extremities of the TI segment(s).

The invention also provides a composition comprising a chimeric immunogen as described herein, and a pharmaceutically or veterinary acceptable excipient. Specific example of such a composition is a vaccine.

Chimeric immunogens of the invention may be used for the treatment of a viral disease, bacterial disease, parasite disease, auto-immune disease, prion disease, inflammatory disease, endometriosis, cancer, or a neurodegenerative disease.

Chimeric immunogens of the invention may also be used in a method for inducing antigen-specific immunoglobulins (such as IgGs, IgMs, IgEs or IgAs, preferably IgGs or a mixture thereof) in a subject, wherein the subject is human or non-human animal, preferably a mammal, e.g., a human, dog, cat, horse, cow or pig; an avian or a fish.

In another aspect, the invention provides a method or use for inducing antigen-specific immunoglobulins in a subject, comprising administering to the subject at least one chimeric immunogen comprising said antigen, or a fragment thereof, coupled to one or more TI segments.

In another aspect, the invention relates to a method or use for vaccinating a subject against a pathogen, comprising administration to the subject of at least one chimeric immunogen comprising an antigen from the pathogen, or a fragment thereof, coupled to one or more TI segments.

The invention further relates to a method or use for stimulating an immune response against an antigen in a subject, comprising administering to the subject at least one chimeric immunogen comprising said antigen, or a fragment thereof, coupled to one or more TI segments.

The invention also relates to a method or use for inducing an anti-IgE immune response in a subject, comprising administering to the subject at least one chimeric immunogen comprising an IgE, or a fragment thereof, coupled to one or more TI segments.

The invention further provides a method or use for neutralizing IgE in a subject, comprising administering to the subject at least one chimeric immunogen comprising an IgE (e.g., autologous or heterologous), or a fragment thereof, coupled to one or more TI segments.

The invention also relates to a method or use for increasing immunogenicity of an antigen, comprising coupling said antigen to a TI segment.

In another aspect, the invention relates to a method of producing a chimeric immunogen (Chimera), comprising coupling an antigen to a TI segment.

In another aspect, the invention also relates to a method or use for stimulating an immune response in a subject against an antigen from a pathogen (such as a virus, bacterium or parasite), comprising administering to the subject at least one chimeric immunogen comprising the antigen from the pathogen, or a fragment thereof, coupled to one or more TI segments.

In another aspect, the invention relates to a method or use for stimulating an immune response against the SARS-CoV-2 antigen preferably selected from Full Length Spike (FLS) protein and five most Exposed Spike domains (5ES).

In a further particular embodiment, the invention relates to a method or use for stimulating an immune response against the S protein of the SARS-CoV-2 in a subject, comprising administering to the subject at least one chimeric immunogen comprising the S protein, or a fragment thereof, coupled to one or more TI segments.

The invention also relates to a nucleic acid molecule encoding a chimeric immunogen as defined above, a vector comprising such a nucleic acid molecule, and/or a recombinant host cell containing such a nucleic acid or vector.

The invention also relates to a composition comprising a chimeric immunogen (or a nucleic acid encoding a chimeric immunogen) and a pharmaceutically or veterinary acceptable excipient..

The invention further provides a method for preventing the development of diseases induced by different pathogens (such as prions, viruses, bacteria, parasites) in human or other mammals or birds or fishes. The invention further provides a method for treating human or other mammals, or birds or fishes, from the development of prion diseases, autoimmune diseases, inflammatory diseases, neurodegenerative diseases, endometriosis or cancers.

The invention may be used with any antigen, such as self-antigens and non-self-antigens, with a human or non-human subject such as any mammal or an avian or a fish.

### Legend to the figures

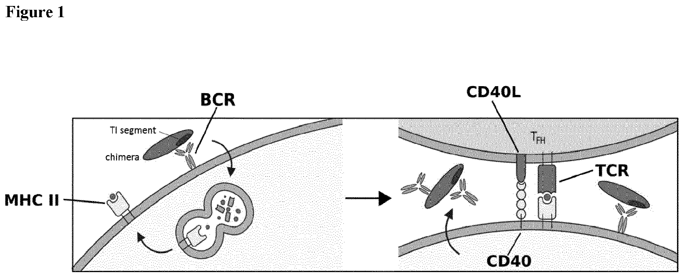

**FIGURE 1****:** Proposed mechanism for TI protein immunogenicity. The B cell receptor (BCR) of naive B cells is composed of natural immunoglobulins (Nlg) located on the surface of the cells in the absence of cognate stimulus.

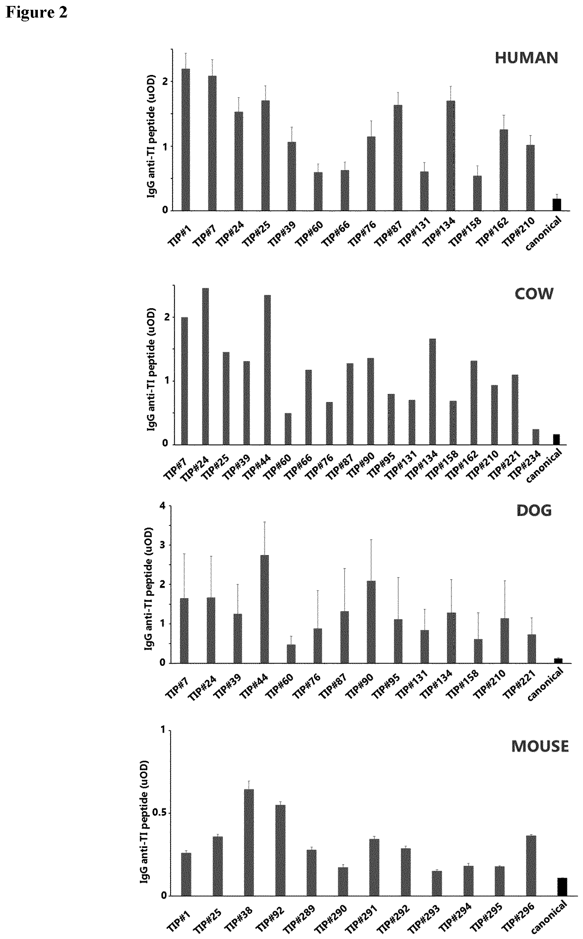

**FIGURE 2****:** Levels of natural IgG present in sera of human, cow, dog and mouse and reactive to a series of TI peptides (TIP) (in red) as enumerated in the below section entitled "Sequences" compared to a canonical peptide (in black).

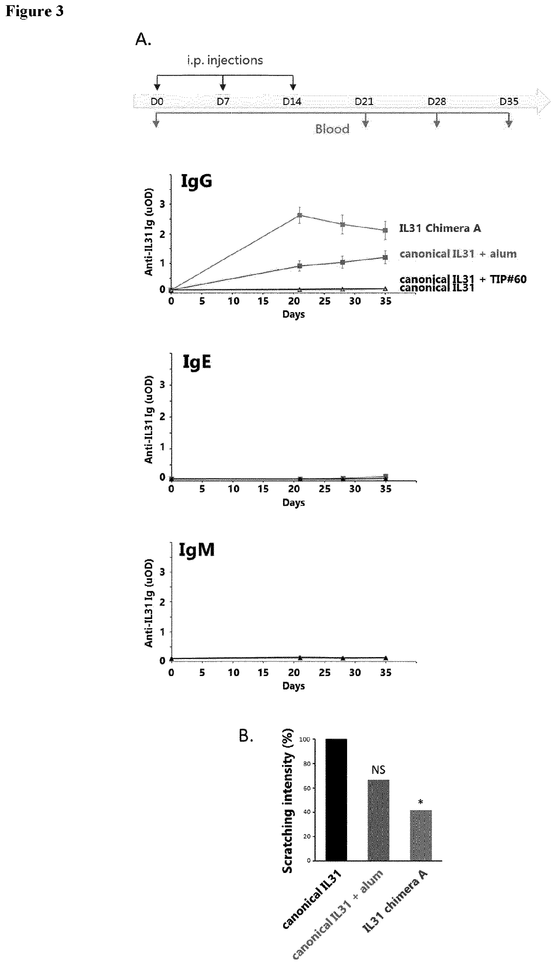

**FIGURE 3****:** Effect of coupling of a TI segment to a non-immunogenic protein on Ig production and scratching in mice. Scheme of the experimental protocol is shown on the top of the Figure. (A) Levels of IgG, IgE and IgM directed against canonical IL31 in mice immunized on days 0, 7, and 14 by injections of 200 mcg of IL31 Chimera A or canonical IL31 with or without alum or canonical IL31 with 20 mcg of TI peptide #60 having a sequence of SEQ ID NO: 21 (expressed as mean +/- SEM). (B) IL31-induced scratching on day 71 after the first immunization of mice with either canonical IL31 or IL31 Chimera A (^{∗}p < 0.05, NS, not significative).

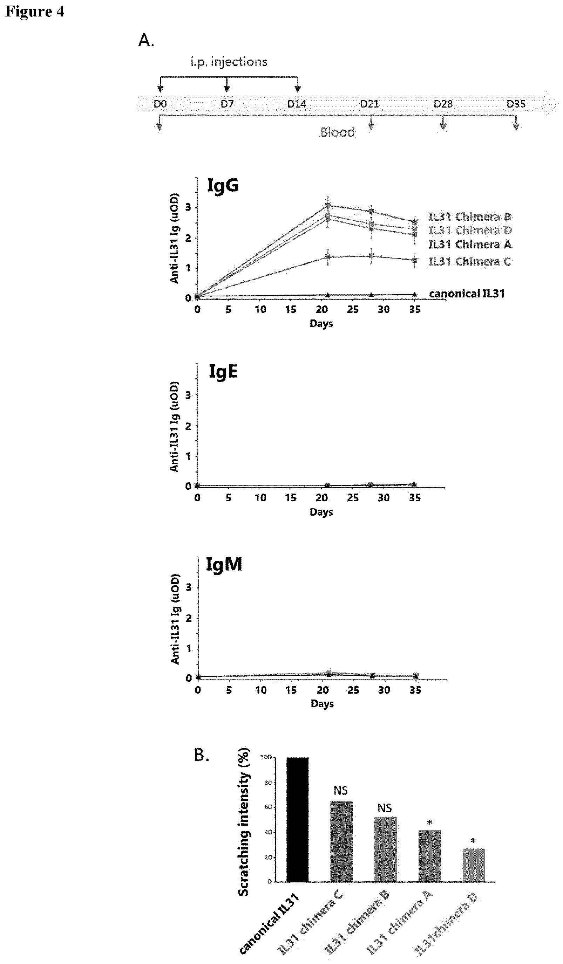

**FIGURE 4****:** Effect of canonical IL31 or IL31 Chimera A-D immunization on Ig production and IL31-induced scratching in mice. Scheme of the experimental protocol is shown on the top of the Figure. (A) Levels of IgG, IgE and IgM to canonical IL31 in mice after 3 injections (days 0, 7, and 14) of canonical IL31 or 4 different IL31 Chimera (expressed as mean +/- SEM). (B) IL31-induced scratching on day 70 after immunization of mice with either canonical IL31 or IL31 Chimera A-D (^{∗}p < 0.05; NS, not significative).

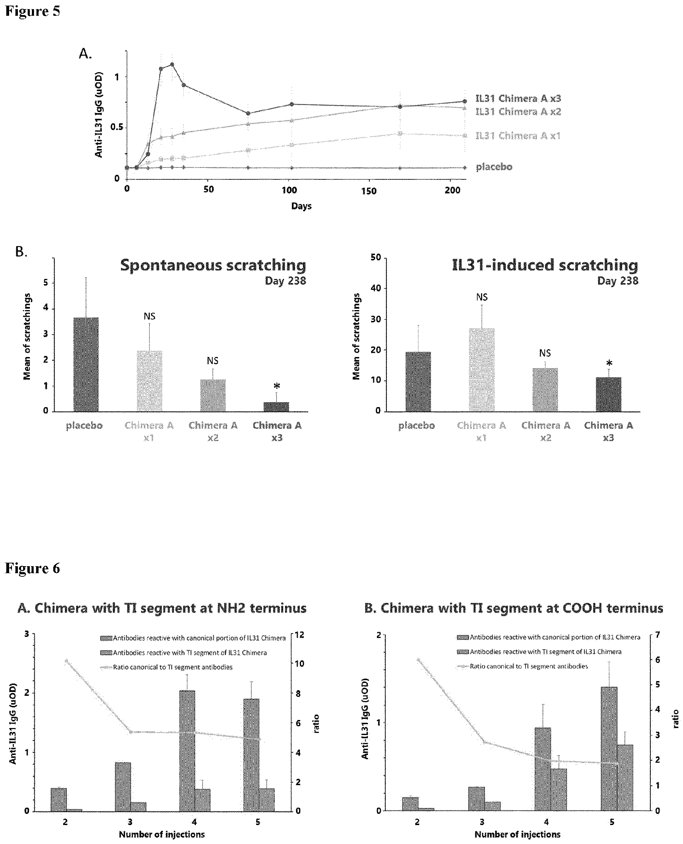

**FIGURE 5****:** Effect of single or multiple injections of IL31 Chimera A in mice on persistent IgG levels and on spontaneous and IL31-induced scratching. (A) Levels of anti-canonical IL31 IgG after 1 (day 0 - IL31 Chimera A x1), 2 (days 0, 7 - IL31 Chimera A x2) or 3 injections (days 0, 7, 14 - IL31 Chimera A x3) of IL31 Chimera A in mice. (B) Spontaneous and IL31-induced scratching on day 238 after immunization of mice with either placebo or IL31 Chimera A, expressed as mean +/- SEM (^{∗}p < 0.05, NS, not significative).
**FIGURE 6****:** Kinetic of induction of IgG antibodies reactive with canonical or TI segments of IL31 Chimera after 2 to 5 injections in mice (days 0, 7, 14, 21, 28), IL31 Chimera being coupled with a TI segment either at its NH2-terminal end (A), or at its COOH-terminal end (B). In A and B, the ratio of antibody reactive with canonical versus TI segments of the target decreases with increasing number of injections (see yellow line). This occurs irrespective of the COOH- or NH2-position of the TI segment.

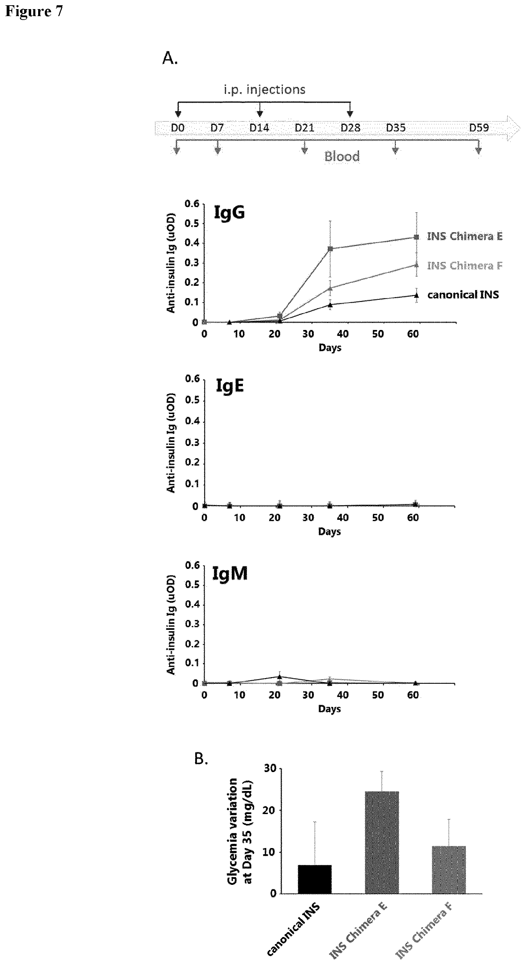

**FIGURE 7****:** Effect of TI segment coupled to a non-immunogenic self-protein, insulin, on Ig production and glycemia in mice. Scheme of the experimental protocol is shown on the top of the Figure. (A) Levels of IgG, IgE and IgM directed against canonical insulin (Canonical INS) in mice immunized on days 0, 14, and 28 by injections of 130 mcg of canonical insulin or insulin Chimera E and F (INS Chimera E & F) (expressed as mean +/- SEM). (B) Variation of glycemia 35 days after immunization of mice with either canonical insulin or INS Chimera E or F.

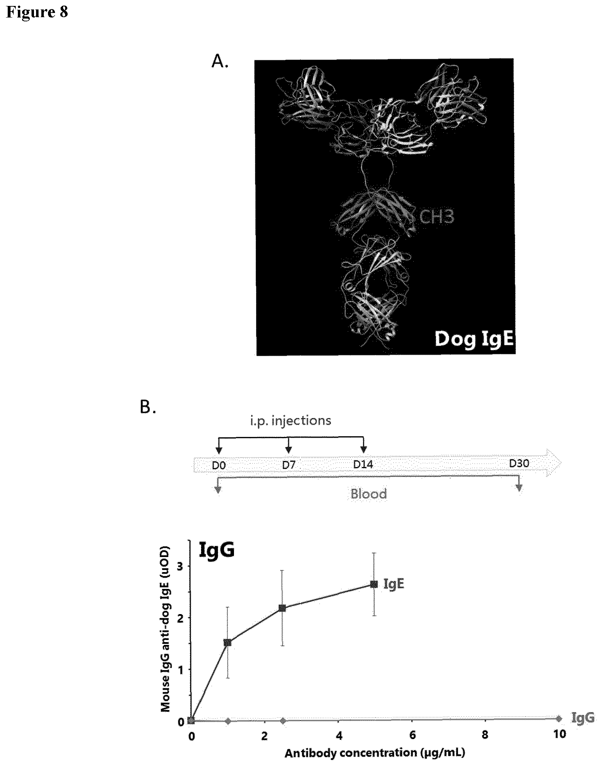

**FIGURE 8****:** Effect of TI segment coupled to an anti-dog IgE-CH3 on IgG production in immunized mice. (A) Computerized three-dimensional model of a dog IgE. The CH3 domain, chosen for design of the IgE Chimera, is shown in red. (B) Scheme of the experimental protocol is shown on the top of the Figure. Levels of IgG reactive to whole dog IgE, and dog IgG detected in mice immunized on days 0, 7, and 14 by injections of 100 mcg of IgE Chimera (expressed as mean +/- SD).

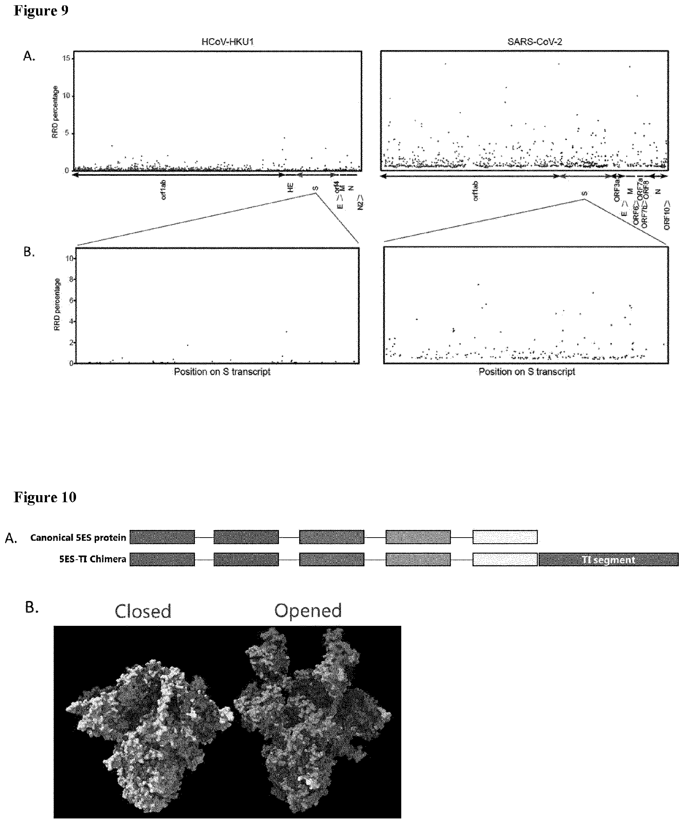

**FIGURE 9****:** Transcription infidelity gap profiles of the pathogenic SARS-CoV-2 and the benign HCoV-HKU1 coronaviruses. (A) Comparison of RNA-RNA divergence (RRD) gap distribution along SARS-CoV-2 and HCoV-HKU1 genomes. Each dot represents a putative TI gap. Position of the sequences encoding for the SARS-CoV-2 proteins are indicated at the bottom of the Figure. (B) Comparison of RRD gap distribution on the S transcripts.
**FIGURE 10****:** Design of SARS-CoV-2 chimeric immunogens. (A) Schematic representation of 5ES construct, derived from S protein of SARS-CoV-2 coronavirus. The putative exposed epitopes are indicated in green, blue, red, grey and yellow; the red epitope is part of the Receptor-Binding Domain (RBD) of S protein. 5ES-TI corresponds to the 5ES coupled to a TI segment. (B) Mapping of these epitopes on the surface of the S protein trimeric complexes in the closed (left) or open (right) conformation. SARS-CoV-2 structures were obtained by a SWISSMODEL prediction based on SARS-CoV structures (Walls, 2019; Song, 2018).

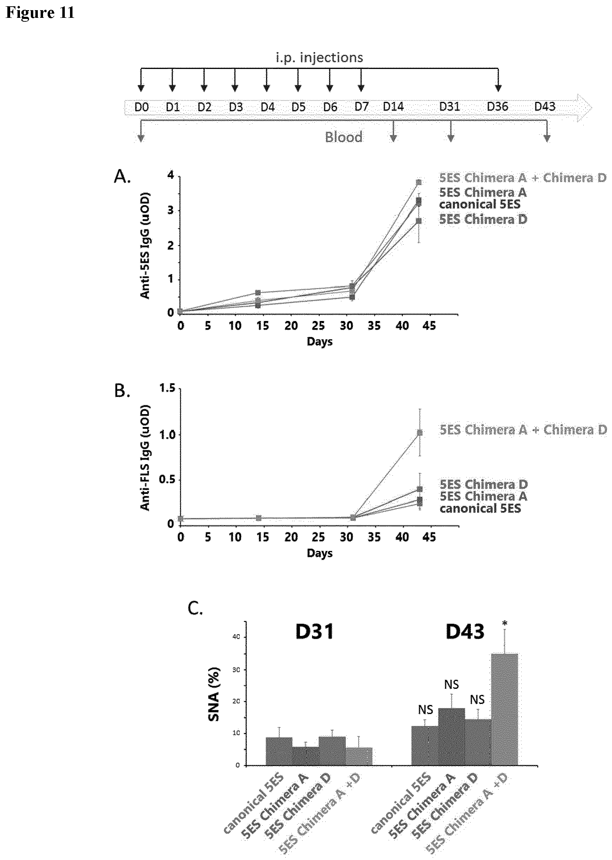

**FIGURE 11****:** Effect of TI-assisted immunization against SARS-CoV-2 coronavirus in mice on Ig production and serum neutralizing activity (SNA). Scheme of the experimental protocol is shown on the top of the Figure. Mice received 5ES Chimera A or 5ES Chimera D or a combination of both (20 mcg of each protein) intraperitoneally (5 mice per group), once a day during 7 days and a single injection (20 mcg of each protein) of the same immunogens on Day 36. Blood samples were collected at the indicated times. The IgG reactivity with 5ES (A) and full length S protein (B), and the percentage of Serum Neutralizing Activity (SNA) (C) were measured (expressed as mean +/- SEM) p-values were calculated with Wilcoxon tests with FDR correction between Day 31 (D31) and Day 43 (D43) (^{∗}p < 0.05, NS, not significative).

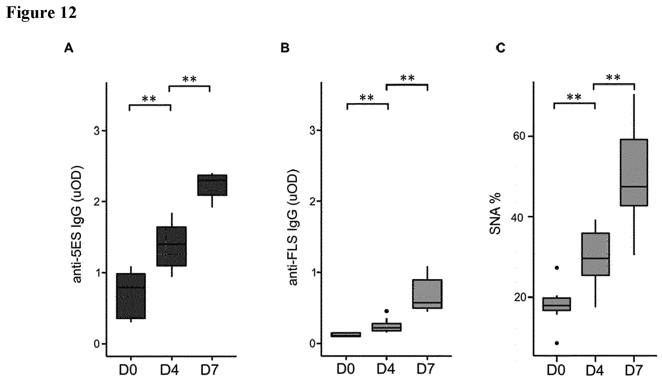

**FIGURE 12****:** Kinetic of induction of SARS-CoV-2 neutralizing antibodies using 5ES-TI A and D. Mice (n=8) were boosted with 5ES-TI A and D (20 mcg each), sera were collected on days 0, 4 and 7. IgG reactive with 5ES (A), FLS (B) and the Serum Neutralizing Activity (SNA) (C) are shown (^{∗∗}p < 0.01).

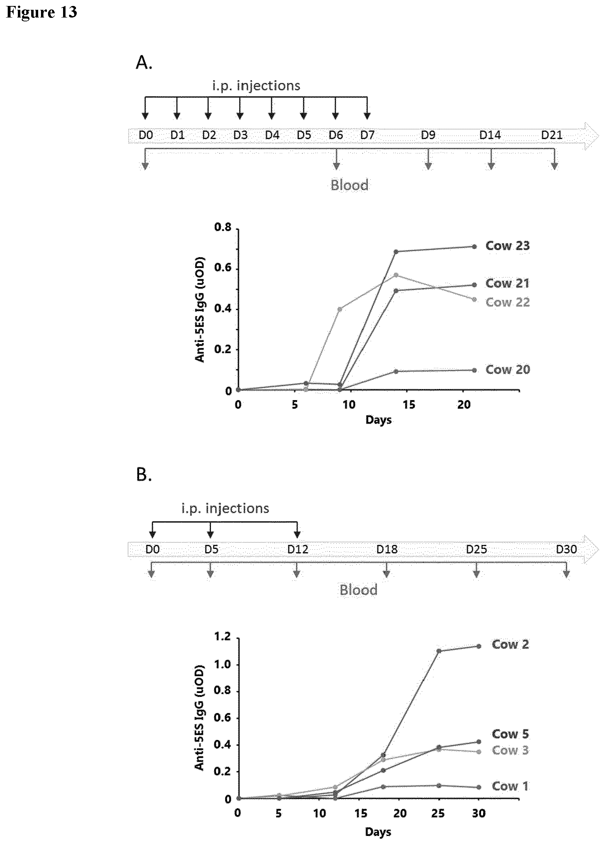

**FIGURE 13****:** Effect of TI-assisted immunization against SARS-CoV-2 coronavirus in cows on Ig production. Scheme of the experimental protocol is shown on the top of the Figure. (A) Cows received the combination of immunogens 5ES-TI A, 5ES-TI B, 5ES-TI C and 5ES-TI D (25 mcg for Cows 20 & 21 or 100 mcg of each protein for Cows 22 & 23) intraperitoneally, once a day during 8 days. Blood samples were collected at the indicated times. The IgG reactivity with 5ES was measured (serum dilution 1:1.000). (B) The combination of the same immunogens (200 mcg of each protein) was injected intraperitoneally, three times at a 1-week interval. Blood samples were collected at the indicated times and the IgG reactivity with 5ES was measured (serum dilution 1:10.000).

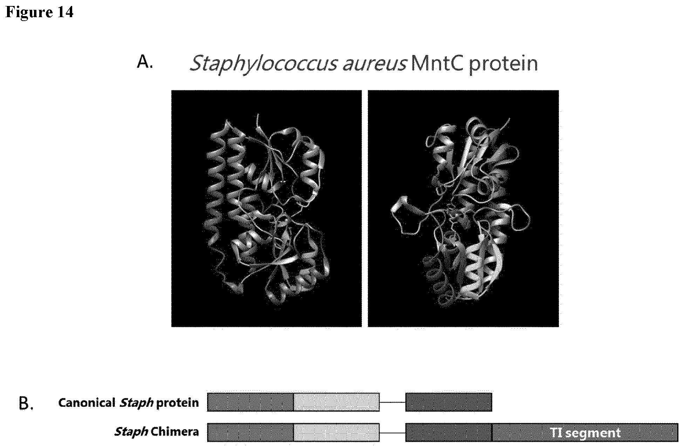

**FIGURE 14****:** Design of *Staphylococcus aureus* chimeric immunogens. (A) Three-dimensional structure of S. *aureus* MntC protein. The highly immunogenic and surface-exposed regions chosen for design of Chimera are shown in red, green and purple. (B) Schematic representation of S. *aureus* canonical (canonical *Staph)* and Chimera (*Staph* Chimera) proteins.

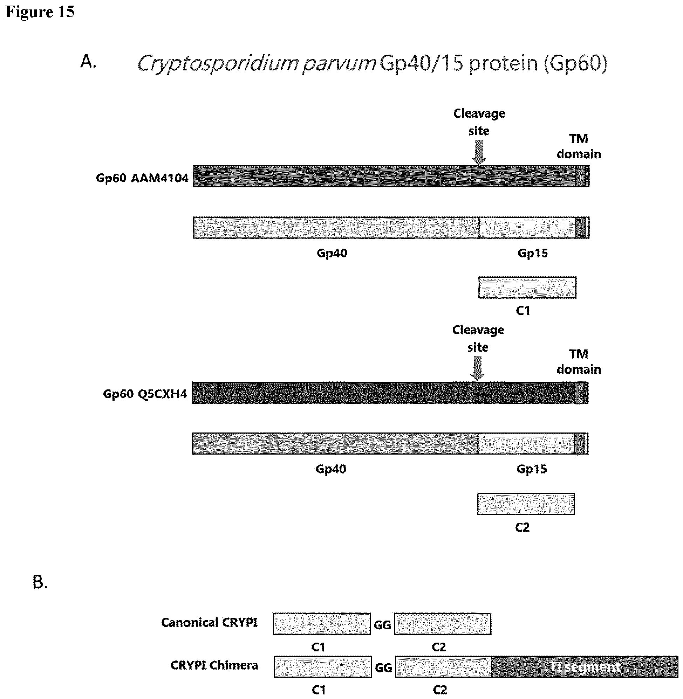

**FIGURE 15****:** Design of *Cryptosporidium parvum* chimeric immunogens. (A) Schematic representation of the two reference gp60 proteins. The transmembrane domain (TM, red box) and the cleavage site leading to gp40 and gp15 formation, as well as the C1 and C2 sequences chosen to construct Chimera are indicated. (B) Schematic representation of C. *parvum* canonical (canonical CRYPI) and Chimera (CRYPI Chimera) proteins.

### Detailed description of the invention

Transcription infidelity (TI) causes RNA to diverge from canonical DNA sequences (Brulliard, 2007; Thouvenot, 2020). TI produces a myriad of low abundance proteins with amino acid (AA) sequences that are different from that predicted by the canonical DNA sequence (Li, 2011). TI has been observed in normal pro and eukaryotic cells, and is increased in cancer cells (Ju, 2011; Li, 2011; Wons, 2015; Brulliard, 2007). TI events may be any base modifications such as substitutions, deletions or insertions. Most TI events are base substitutions but TI also causes single base gaps and the resulting frameshifted RNA translate into proteins with a carboxyterminal segment that generally becomes cationic. The resulting TI proteins afford specific immunological properties (Thouvenot, 2020).

Here we show that such TI segments have immunological properties and can confer on the translated as well as on other molecules (e.g. proteins) of interest, the capacity to induce the production of antibodies without the need for any adjuvant. We further show that the isotype of these antibodies as well as their affinity can be determined by the amino acid (AA) composition of the TI segment. We also show in the present invention that the resulting antibodies react with both the TI and the canonical portion of the eliciting chimera. The invention thus provides a novel, unique and potent approach to elicit antibodies directed towards any antigen, including thus far non immunogenic proteins, irrespective of their origin from self or non-self.

An overall higher RNA-DNA divergence (RDD) rate is observed in *Cryptosporidium* versus *Trypanosoma Cryptosporidium* is a parasite controlled by the normal immune system hence only emerges when the latter is defective, e.g. newborn or immunocompromised patients while *Trypanosoma* on the other hand is far less immunogenic. The same inverse association between RDD rate and pathogenicity is observed in 2 distinct bacterial strains. Indeed the overall RDD rate is lower in *Mycobacterium tuberculosis* versus BCG strain, and is also lower in anthrax versus *Bacillus subtilis.* Considering that TI defines the repertoire of natural immunoglobulins, we propose that pathogenic bacteria, in contrast to their less pathogenic counterparts, have reduced RDD rate thereby providing a stealth mode to escape immune detection. However, their overall stealth strategy is not absolute thereby revealing novel points of weaknesses that can be immunologically targeted by the technology disclosed here. Indeed, specific TI events are increased in these various pathogens relative to their less pathogenic counterparts, corroborating the bioinformatic analysis of different coronavirus genomes.

TI rates occurring in pathogenic *Escherichia coli* are also higher than those observed within the same DNA context in their less pathogenic counterparts. Therefore, the method disclosed here enables one skilled in the art to identify the site of vulnerability of any pathogens in spite of its overall stealth mode, then select the most appropriate targets and produce chimeric immunogens (such as proteins) eliciting immune response toward said targets thereby inducing the appropriate antigen-specific immunoglobulins targeting specifically pathogen sites of vulnerability. These chimeric immunogens may be produced in the form of preventive vaccines that will for example reduce *Pseudomonas aeruginosa* burden in patients with cystic fibrosis patients or with Chronic Obstructive Pulmonary Disease. These differential TI segments provide novel probes allowing to delineate how effective immune response to these pathogens is naturally produced by most individuals but far less efficient in others, most commonly children and the elderly. Identification of differences in natural Ig repertoire associated with better and poorer clinical outcome to infectious diseases also provides a novel strategy needed to improve overall vaccine efficacy in specific population.

### Chimeric Immunogens

"Chimeric immunogens" or "chimera" according to the invention are immunogens which comprise (i) one or more antigen(s), or fragment(s) thereof, which are coupled to (ii) one or more transcription infidelity (TI) segments. Chimeric immunogens are preferably produced by covalent coupling of target antigen(s) of interest with selected TI peptidic segment(s) having a specific AA composition. Such immunogens are capable of inducing humoral and/or cellmediated immune response.

The invention shows that immunization with a chimeric antigen comprising a TI segment and an antigen of interest leads to a potent humoral response. Such finding has been demonstrated by the inventors with foreign antigens (e.g. non-self-antigens) as well as with self-antigens (such as IL31 or insulin).

Within the context of the invention, coupling between antigen(s) and TI segment(s) is preferably covalent. Coupling may be performed directly between the TI segment and the target antigen, i.e. though a direct chemical bonding (or genetic fusion) between the two or more entities, or indirectly through a linker group.

In a particular embodiment, where the antigen is a protein or polypeptide, coupling is through a peptide bond, which may be obtained by genetic fusion (e.g. expression of a recombinant gene encoding the two or more entities as a chimeric protein) or by chemical reaction.

Coupling may be performed with any suitable position/residue on the segment and antigen. Coupling can be at one or more N-terminal and/or C-terminal ends of the TI segment and/or the antigen, as well as at any internal reactive group of the TI segment and the antigen, such as reactive groups carried by internal AA side chains or any posttranslational decoration. Coupling can be carried out at any site of the segment or antigen where functional groups such as -OH, - SH, -CO2H, -NH2, -SO3H or -PO2H are naturally present or have been introduced. Means of covalent chemical coupling, calling upon a spacer or not, include those selected from bi- or multifunctional agents containing alkyl, aryl or segment groups by esters, aldehydes or alkyl or aryl acids, anhydride, sulfhydryl or carboxyl groups, groups derived from cyanogen bromide or chloride, carbonyldiimidazole, succinimide esters or sulphonic halides.

In a particular embodiment, the chimeric immunogen comprises one antigen, or a fragment thereof, coupled to one TI segment. In a particular embodiment, the chimeric immunogen has the structure A-P or P-A, where A is the antigen part of the molecule and P is the TI segment. Coupling is preferably terminal. The TI segment can also be inserted inside the antigen and the chimeric immunogen has the structure A1-P-A2, where A1 and A2 are the two parts of the antigen A and P is the TI segment.

In a further particular embodiment, the chimeric immunogen comprises one antigen, or a fragment thereof, coupled to two TI segments or more. In such constructs, the two or more TI segments may be the same or different. Also, they may be conjugated at different positions on the antigen, or on the same position. Accordingly, in a particular embodiment, the chimeric immunogen has the structure Pn-A-Pn, or A-P-Pn, where A is the antigen part of the molecule, P is a TI segment, and n is an integer from 1 to 5. In other embodiments, the chimeric immunogen may comprise two or more antigens.

In a preferred embodiment, the antigenic element is a polypeptide or protein, and the chimeric immunogen comprises the antigenic element and the TI segment directly covalently coupled by genetic fusion.

The chimeric immunogen may be prepared by methods known *per se* in the art, such as by recombinant techniques, or chemically, or enzymatically, or through a combination of these techniques. The chimeric immunogen may be purified, concentrated, placed in solution, or lyophilized, or frozen. The chimeric immunogen may also be generated *in situ* by gene modification by methods known *per se* in the art, such as editing.

Chimeric immunogens according to the invention may also be tagged. All protein tags known in the art are suitable for tagging of chimeric immunogens according to the invention. Such tags include for example a His tag (polyhistidine), e.g., HHHHHH, MKHHHHHH, a FLAG tag (DYKDDDDK), a GST tag, a MBP tag or a Myc tag, which may be fused to proteins of interest using any expression vector systems known in the art. The tags may be placed at the N-terminal or at the C-terminal end of the protein.

Specific examples of chimeric immunogens according to the invention are chimeric immunogens comprising or consisting of a sequence of any of SEQ ID NO: 2, 52, 3, 53, 4, 54, 5, 55, 6, 56, 7, 57, 9, 59, 10, 60, 12, 62, 14, 64, 15, 65, 16, 66, 17, 67 (as shown in the section "Sequences" and in the experimental section).

### TI segment

The term "TI segment" designates, within the context of this invention, a stretch of amino acids (or an amino acid sequence) comprising, consisting essentially, or consisting of a sequence or a portion of a sequence resulting from a transcription infidelity (TI), or a variant thereof. More particularly, TI segment designates any TI amino acid sequence resulting from TI in any protein, wherein TI may be any base modification such as a substitution, deletion or insertion. TI segments may be selected from any TI segment of any proteins generated through TI, namely any human or non-human animal proteins generated through TI but also any microbial proteins, e.g., bacterial or viral protein, generated through TI. Alternatively, methods of isolating such proteins, or for artificially conceiving such TI segments, have been disclosed by applicants, e.g. in WO2008/009751. From such methods, TI segments can be identified, isolated, and/or artificially synthesized. In particular embodiments, TI segments according to the invention have a specific amino acid composition which is enriched in basic amino acids and/or depleted in acid amino acids. In other specific embodiments, TI segments according to the invention have an isoelectric point (pl) of 7.4 or more, 7.6 or more, 7.8 or more, preferably 8 or more, 8.5 or more, or 9 or more. In other specific embodiments, TI segments according to the invention have (i) a specific amino acid composition enriched in basic amino acids and/or depleted in acid amino acids, and (ii) an isoelectric point (pl) of 7.4 or more, 7.6 or more, 7.8 or more, preferably 8 or more, 8.5 or more, or 9 or more.

TI segments according to the invention typically contain between 3 to 70 amino acids in length, particularly from 5 to 50, from 5 to 40, from 5 to 35, or from 10 to 50, from 10 to 40 or from 10 to 35. The terms "TI segment" and "TI peptide" are interchangeable.

The invention uses any TI segment as described herein, and preferably any TI segment selected from TI segments enumerated in the section below entitled "Sequences". Examples of TI segments from human or non-human animals, or from microbes, are shown in the experimental section and in the section "Sequences". In particular embodiments, TI segments according to the invention comprise, consists essentially of, or consist of a sequence selected from any of SEQ ID NOs: 20 to 50. Examples of TI segments common to human and animal species are also disclosed in the experimental section.

For preparing a chimeric immunogen of the invention, any such TI segments can be used, and coupled to any antigen of interest. Depending on the nature of the response that needs to be elicited, it may be possible to select most appropriate TI segments.

The term "TI proteins" as used herein, designates any proteins comprising TI segments. TI protein may be a naturally occurring protein or an artificially constructed chimera constituted of a complete or partial target protein fused with a selected TI segment with a defined amino acid composition. Both types of TI proteins have been shown to be immunogenic. In naturally produced TI proteins, the TI segment is always on the carboxyterminal end, while in constructed chimera, it can be placed at either C-terminal extremity or N-terminal extremity or at both extremities or within the canonical portion of the protein.

### Antigen

The invention may be used with any antigen, including self- and non-self-antigens. The antigens are preferably protein antigens. The self-antigens are human or non-human animal antigens (mammalian or avian, fish). The non-self-antigen may be selected from prion, viral, bacterial, parasite or mammalian proteins. In particular embodiments, the antigens according to the invention are human endogenous proteins such as PCSK9, LPA, CETP, LSR agonist, Ghrelin, Amyloid β, α-synuclein, insulin, chemokines, cytokines e.g. IL31, IL33, TSLP, immunoglobulins e.g. IgE, etc., or their equivalents in non-human mammals (e.g. a cow, pig, horse, dog, cat, etc.)

In another particular embodiment, the antigen according to the invention is a prion, viral, bacterial or parasite protein infecting humans and/or non-human animals such as mammals (e.g. cows, pigs, horses, dogs, cats) or birds or fishes. Preferably, the viral antigen is any suitable antigen from infectious viruses such as human immunodeficiency virus (HIV), Hepatitis A, B, C, D or E, papillomavirus, coronavirus, Epstein-Barr virus (EBV), Influenza virus A, B, C or D. Other viral antigens according to the invention are porcine viruses (such as porcine respiratory virus), etc. In this regard, in a particular embodiment, the vaccines intended for piglets or veals should be delivered to pregnant sows or cows in order to facilitate the immunization process using at least three injections without adjuvant, thereby resulting in colostrum strongly enriched in specific Ig. In a preferred embodiment, the viral antigen according to the invention is a coronavirus antigen, preferably, an antigen from SARS-CoV-2. Preferably, the SARS-CoV-2 antigen is preferably selected from Full Length Spike (FLS) protein and five most Exposed Spike domains (5ES). The bacterial antigen according to the invention is any antigen from infectious bacteria, such as *Mycobacterium tuberculosis, Pseudomonas aeruginosa, Escherichia coli, Bacillus subtilis, Bacillus anthracis, Staphylococcus aureus, etc.*

This would be particularly relevant for multidrug resistant bacteria. The preferred parasite antigens are proteins from *Cryptosporidium* (such as *Cryptosporidium parvum*)*, Trypanosoma* (such as *Trypanosoma brucei*)*, Plasmodium,* etc.

In another embodiment, the target antigen according to the present invention may also be any protein against which is directed an autoimmune antibody secreted in any autoimmune disease (such as Type I diabetes, Parkinson disease, Hashimoto hypothyroidisms, etc.) or other diseases such as a cancer or endometriosis. The term "antigen" includes full length antigenic protein or a fragment thereof. The term "protein" designates a molecule comprising AAs, and designates polypeptides, proteins, or peptides, which may be of natural origin, purified, modified, recombinant, synthetic, carrying or not any type of post-translational modifications.

### Pharmaceutical compositions, vaccines

In a further embodiment the invention relates to a composition comprising a chimeric immunogen as described herein, in combination with one or more pharmaceutically or veterinary acceptable carrier(s), excipient(s), sprays, or aerosols, thus forming a pharmaceutical or veterinary composition.

Preferably, such a composition is for use in the treatment of a viral disease, bacterial disease, parasite disease, auto-immune disease, prion disease, inflammatory disease, endometriosis, cancer, or a neurodegenerative disease. Preferably, such a composition is a vaccine.

The composition may be in the form of, e.g. tablets, capsules, pills, powders, granulates, suspensions, emulsions, solutions, gels including hydrogels, pastes, ointments, creams, plasters, drenches, osmotic delivery devices, suppositories, enemas, injectables, implants.

### Modes of administration

Within the context of the present invention, the chimeric immunogens or compositions, as described herein, may be administered to the subject by any suitable route of administration such as parenteral (e.g. intravenous, intramuscular), oral, nasal, rectal or cutaneous route. Preferably, the chimeric immunogens or compositions according to the invention, are administered by injection, e.g. sequential injections.

In a preferred embodiment, the chimeric immunogens or compositions according to the invention, are administered sequentially, at least three times, at 1 to 15 days intervals, during one, two, three months, or more.

### Methods and uses of the invention

The methods and uses described here can in fact elicit production of antibodies of the desired isotype and with the desired affinity directed against any protein, with/without any post-translational modification, e.g. glycosylation, and originating from self or non-self. Currently, it is commonly not possible to elicit production of clinically meaningful antibodies targeting self-proteins without co-administration of strong adjuvant. The method of the present application is capable of eliciting oligo clonal antibodies that have the ability to effectively block the function (i.e. have sufficient affinity) of a cytokine operating in the nanomolar range. The titer of immunoglobulins (such as IgG) elicited in response to chimeric immunogens of the invention can persist at least 6 months, or even 12 months (as shown by the inventors after mouse immunizations) and even longer after the injections. If appropriate, anti-idiotypic antibodies directed toward the specific paratope recognized by TI Chimera antibodies can be elicited by the same technology thereby neutralizing the vaccine (and in monoclonal-induced autoimmune disease if any).

Understanding the mechanism that cause important or less important T cell development is of key in the design and elicitation of antibodies capable of removing peptides/proteins that are associated to pathological conditions because of their accumulation in specific tissues, e.g. Aβ amyloid forming plaque that contributes to Alzheimer pathology or alpha-synuclein that contributes to Parkinson disease pathology. It has indeed been observed that vaccine eliciting Aβ specific T cell caused significant cellular toxicity and aseptic meningo -encephalitis in 6 % of vaccine recipients.

In a first embodiment, the invention provides a method or use for inducing antigen-specific immunoglobulins in a subject, comprising administering to the subject at least one chimeric immunogen comprising said antigen, or a fragment thereof, coupled to one or more Transcription Infidelity (TI) segments. Preferably, the coupling between said antigen and one or more TI segments is covalent, through a peptide bond, direct or through a linker group, either at COOH-terminal extremity or NH2-terminal extremity, or at both extremities of the TI segment(s).

The method of the invention may comprise several sequential administrations of a same or different chimeric (s) immunogen(s).

In a further embodiment, the immunoglobulins induced according to the invention, are IgGs, IgMs, IgEs or IgAs, preferably IgGs or a mixture thereof. In a preferred embodiment, the IgGs have an affinity of 10E-9 or 10E-10 M or less.

The above methods or uses for inducing antigen-specific immunoglobulins in a subject, according to the invention, may further comprise a step of collecting a serum sample from said subject, said serum sample comprising antigen-specific immunoglobulins. Said serum sample may be from any subject, e.g., a human subject or a non-human animal such as a mammal e.g., a dog, cat, horse, cow or pig; or an avian or a fish. For example, said serum sample may comprise anti-SARS-CoV-2 specific immunoglobulins. In a particular embodiment, such anti-SARS-CoV-2 specific immunoglobulins may be used for the treatment of Covid-19 by serotherapy.

The methods and uses of the invention may be applied to human subjects and to non-human subjects, preferably mammals such as dogs, cats, horses, cows or pigs; or avians, or fishes.

The invention also provides a method or use for prophylactically or therapeutically vaccinating a subject against a pathogen, comprising administering to the subject at least one chimeric immunogen comprising an antigen from the pathogen, or a fragment thereof, coupled to one or more TI segments.

In a particular embodiment, the invention provides a method or use for prophylactically or therapeutically vaccinating a subject against a SARS-CoV-2 pathogen, comprising administering to the subject at least one chimeric immunogen comprising an antigen from the SARS-CoV-2 pathogen selected from Full Length Spike (FLS) protein and five most Exposed Spike domains (5ES), or a fragment thereof, coupled to one or more TI segments. As shown with current mRNA SARS-CoV-2 vaccines, humoral response induced using such a method would allow to bring down Covid-19 conversion to severity.

The invention also provides a method or use for inducing an anti-IgE IgG immune response in a subject, comprising administering to the subject at least one chimeric immunogen comprising an IgE, or a fragment thereof, coupled to one or more TI segments.

The invention also provides a method or use for neutralizing IgE in a subject, comprising administering to the subject at least one chimeric immunogen comprising an IgE, or a fragment thereof, coupled to one or more TI segments.

The invention also relates to a method or use for increasing immunogenicity of a molecule, comprising coupling said molecule to a TI segment.

The invention also provides a method for selecting TI segments that are susceptible to be fused to self or non-self proteins, or fragments thereof, in order to generate TI proteins able to elicit strong humoral response.

The invention also provides a method for comparing TI repertoires of pathogenic vs non-pathogenic organisms that allows to select candidate proteins for chimerization.

The invention also provides a method for identifying patients susceptible to be treated with a specific TI protein.

The invention also provides a method for producing novel animal models to dissect and analyze autoimmune disorders, allowing to select candidate proteins for chimerization and for the treatment of autoimmune disorders according to the method of the present invention. The present invention provides novel molecules (chimeric immunogens) able to break self-tolerance with minimal T cell involvement. Other endogenous self-proteins can be removed/inhibited by the technology described here.

Using sequential immunization with a single or a cocktail of chimeric immunogens, it is possible to induce affinity maturation of induced antibodies. We have shown that natural immunoglobulins are poly-reactive constituting a network of antibodies that react with more or less closely related TI peptides dependent of the repartition of their high density of positively charged residues. The correlation established between the different TI peptides defines how closely related TI peptides actually are. This discovery enables 2 novel strategies to modulate not only the isotype of elicited antibodies but also the affinity of said antibodies. By constructing a chimeric immunogen according to the invention, an immune reaction biased toward a specific immunoglobulin isotype can be elicited. One can next cause affinity maturation by sequential injection with different Chimera cross reacting with said initial antibody. This process can be readily applied to any target in any species provided that its specific network of natural antibodies has been identified using the TI probes disclosed herein.

The invention thus allows to elicit primarily a specific isotype that is deemed most effective to address specific pathology. Indeed, if in most clinical circumstances, it will be suitable to elicit an IgG response, it is also possible to elicit production of IgM if the target is of a size sufficient to allow multiple IgM binding site that by their combined avidity may favor clearance. In other embodiments, such as cancer immune-surveillance, where one wishes to prevent recurrence of very small amounts of cancer cells after complete elimination of primary tumor and metastasis, eliciting very high affinity IgG or even IgE might be deemed more suitable.

In a preferred embodiment, the induced immunoglobulins are IgGs, preferably IgGs having an affinity of 10E-9 M or less. In another preferred embodiment, chimeric immunogens of the invention elicit both IgG and IgE.

Remarkably, the invention shows that, depending on the amino acid (AA) composition of TI segments, the nature (e.g. IgG or IgM, etc.) and strength (e.g. antibody affinity) of the immune response can be determined and oriented. In this respect, the invention shows the AA composition determines the capacity of the chimeric agents to react with natural immunoglobulins (Nlgs) that are more or less effectively activated and elicit specific isotype switching. The discovery reported here, that TI segments and TI proteins cause the production of Nlgs and share the common properties of being cationic, provides a simple explanation to the finding that natural immunoglobulins are poly-reactive. The data presented here document this poly-reactivity in different ways: Ig reactivity with different TI segments, correlated more or less with one another, thereby establishing a network of poly-reactive immunoglobulins. The explanation for the poly-reactivity lays in the fact that negatively charged AA in the paratope of Nlgs react with more or less densely organized positive charges present in basic AA because of the frameshift accumulated on the TI proteins. We further demonstrate herein a specific application of the discovery of the molecular cause of Nlg polyreactivity by using the proximity of different TI segments coupled to the same target protein to obtain through sequential immunization specific antibodies that progressively become more and more affine with the target through directed affinity maturation. Such broadly neutralizing antibodies may be generated against invariable regions of prions, viruses, bacteria, parasites, mammalian, fish or avian proteins. The capacity to elicit antibodies specifically targeting these invariable domains that are not naturally immunogenic for most people afford important opportunities in the development of universal vaccines for infectious diseases such AIDS or related diseases, flu, tuberculosis, malaria, sleeping sickness and Chagas disease, Covid-19 as well as diseases caused by pathogens that have thus far resisted conventional vaccination protocols. The invention can also be used to produce novel animal models of autoimmune disorders and dissect at the cellular level the interplay between humoral and cellular immunity. The TI segments and the immunization protocol disclosed herein provide the necessary probes that do not require any adjuvant but will produce very high affinity antibodies that can mobilize other components of innate immunity, i.e. complement and/or macrophage, or associate with specific T cells to cause destruction of specific tissues. Furthermore, specific TI proteins generated by endogenous tissues in the context of specific genetic background and eventually triggered by infectious agents (such as viruses) may naturally cause the production of self-destructive auto-antibodies involved in causation of autoimmune diseases, e.g. Type I diabetes, Parkinson disease, Hashimoto hypothyroidisms. TI segments described herein provide novel molecular probes to more rapidly detect the occurrence and progression of auto-immune self-destruction.

In summary, the technology described here, for the first time, enables by-passing the typical restriction of antibody production toward self-proteins. We propose that Chimera and TI proteins unlike conventional adjuvant directly target the antigen to naive B cells decorated by B cell receptor (BCR) containing primarily Natural Immunoglobulin (Nlg). By selecting TI segments recognized primarily by NlgG preferentially to natural IgM, one elicits oligo clonal affinity maturation of IgG when TI segments have adequate AA composition. These affinity matured IgG acquire the desired affinity towards the self-protein target. Alternatively, if the TI segment has a distinctive AA composition than both affinity maturation and isotype, switching occurs leading to production of IgE.

More particularly, TI proteins are immunogenic because they react specifically through their TI segment with Nlg that constitute the BCR of naive B lymphocytes. This leads to presentation by the Major Histocompatibility Complex Class II (MHCII) of TI protein fragments resulting in the engagement of the T cell receptor (TCR) with the assistance of co-stimulatory molecules such as CD40 (as detailed in Example 1 and in Figure 1).

Nlg, unlike cognate immunoglobulin, are poly-reactive (Jordan, 2013; Balakrishnan, 2011). This promiscuity was difficult to explain initially at the molecular level but has become much simpler since the inventors have established that Nlg ligands are TI variants that share the common characteristics of being cationic. Nlg are poly-reactive which means that they can engage any TI proteins or Chimera albeit with differential affinity. This affinity is determined by the AA composition of TI segment. Only BCR with high affinity for a specific TI segment are engaged when low amounts of TI proteins are injected. Inventors estimate that high affinity immunoglobulins obtained after at least 3 injections of Chimera, are able to effectively block 90 % of the function of a cytokine operating at 10⁻⁹ M, and must have a K_{D} < 10⁻¹⁰ M. Assuming that a log of affinity is gained at each round of injection, initial naive BCR affinity being estimated at 10⁻⁷ M. However, one must also expect that engaging a larger panel of B cells with lower initial affinity (10⁻⁴ to 10⁻⁶ M) through injections of high doses of TI proteins yields antibodies with higher titers but much lower affinities, hence less efficient in blocking the activity of the target.

The invention may be used for inducing antigen-specific immunoglobulins in a subject in need thereof, for example in a subject having an infectious disease, an autoimmune disease, inflammation, cancer, endometriosis or a neurodegenerative disease.

In a particular embodiment, the proposed mechanism of TI protein immunogenicity according to the invention has an important role for TI guided vaccination programs, by using the TIASI (TI-assisted sequential immunization) technology. Indeed, it is generally indispensable to screen the immune response in available rodents that will not in most cases be the intended commercial target. The inventors are now able to measure in the intended species the Nlg repertoire and, as a result, rationally select the TI segments with the highest affinity naive BCR and, most importantly, the lowest number of intermediate and low affinity BCR. The inventors, at present, are producing a diversified portfolio of disruptive therapeutic and preventive vaccines addressing several unsolved issues in human and animal health (e.g. *Trypanosomes, Cryptosporidium* and African swine fever). Further, commercial products will be either solubilized injectable proteins or protein powders to be reconstituted. In both cases, the products are highly stable and, unlike common vaccines, do not require cold storage nor co-administration with adjuvant.

In a preferred embodiment, the methods and uses according to the invention are implemented without adjuvant. However, the methods and uses of the invention may also be used with any type of adjuvant.

### Examples

### EXAMPLE 1: Proposed mechanism of TI protein immunogenicity

The inventors previously showed that 1/ transcription infidelity is a natural phenomenon increased under certain conditions like cancer; 2/ in response to TI proteins generated by cancer, human and mice produced IgG (so-called TIAB for Transcription Infidelity AntiBodies) directed to the TI segment of TI proteins; 3/ although highly increased in cancer, these TIAB are naturally produced in the absence of cancer, contributing to the diversity of natural immunoglobulin repertoire.

Hence the inventors propose that TI proteins are immunogenic because they react specifically through their TI segment with Natural Immunoglobulins (Nlg) that constitute the B cell Receptor (BCR) of naive B lymphocytes (Figure 1). This leads to presentation by the Major Histocompatibility Complex Class II (MHCII) of Chimera fragments resulting in the engagement of the T cell receptor with the assistance of co-stimulatory molecules, e.g. CD40 (Figure 1). Endosomal processing of TI proteins therefore starts at the site opposite to that of the TI segment engaged with hence protected by the BCR. This explains that the antibodies directed toward the non TI segment of the TI proteins arise before fewer injections than those reactive with the TI segment (see Example 4). This occurs irrespective of the position of the TI segment at the COOH or NH2 terminal end of the TI protein. Antibodies reactive with the TI segment would be produced only when novel BCR, no longer on naive but on mature B cell, have been produced. These mature B cells could produce BCR specific of the canonical portion of the TI protein. Most importantly, these observations can be used to increase affinity maturation of antibodies directed toward the intended target by first breaking the tolerance to self with a TI protein injection followed by injections of only the canonical protein target.

These observations are consistent with the proposed mechanism of TI protein immunogenicity (Figure 1). Indeed, the TI segment engaged with the BCR in the endosome is likely less accessible to proteases and therefore yields a lower amount of segments to be loaded on MHCII. Antibodies reactive with the TI portion would be produced only when novel BCR, no longer on naive but on mature B cell, have been produced. These mature B cells could produce BCR specific of the canonical portion of the TI protein.

### EXAMPLE 2: Selection of TI segments to be coupled to obtain Chimera for TI-assisted immunization

As previously explained, the inventors propose that TI proteins are immunogenic because they react specifically through their TI segment with Natural Immunoglobulins (Nlg) that constitute the B cell Receptor (BCR) of naive B lymphocytes. Hence, exploring the repertoire of natural Ig of the organism in which a TI-assisted immunization is planned means to identify the best TI segment(s) to be coupled to the immunization target.

Immobilized Nlg on B cells also exist as circulating molecules in sera of different mammalian species. A large panel of segments resulting from TI has been constituted by the inventors through previous work (disclosed for instance in WO2008/009751). The inventors tested in the sera of 4 different species (mouse, human, dog, cow), the reactivity of Nlg with a representative panel of TI peptides having a sequence selected from any of SEQ ID NO: 20 to 50 (as enumerated in the section entitled "Sequences") versus a canonical control peptide, i.e. a peptide encoded by RNA without frameshift. For this experiment, streptavidin plates were coated with biotinylated TI peptides (0.5 to 1 µM per well) and incubated with diluted sera for 30 min at 37°C. After washing, TI peptide-specific IgG were detected by alkaline-phosphatase-labeled anti-human, dog, cow or mouse IgG and development was performed with p-nitrophenyl phosphate (pNPP). The reactivities of Nlg from the 4 mammalian species to TI peptides were significantly higher than those to the canonical peptide (Figure 2). The presence of detectable IgG against TI peptides in sera gives an image of the nature of the Nlg immobilized on B cells. Consequently, TI peptides leading to high level of TIAB should be of better chance to constitute good anchors for B cell receptors of naive B cells and would like to maximize the TI-assisted immunization process.

### EXAMPLE 3: Addition of a cationic TI segment to the carboxy-terminal end of a non-immunogenic protein is sufficient to elicit the production of specific and neutralizing IgG in immunized mice

IL31 is an inflammatory cytokine known as a key player in pruritus and inflammation in atopic dermatitis. Blocking its action, e.g. by using monoclonal antibody as nemolizumab, results in a reduction in pruritus (Silverberg, 2020).

In this experiment, a chimeric protein has been generated by coupling various TI segments having a sequence of SEQ ID NO: 21 (of the section entitled "Sequences") to the carboxyterminal end of IL31 protein. This Chimera (IL31 Chimera A) and the canonical IL31 protein were produced as recombinant N-terminal-hexa-histidine-tagged proteins in *E. coli* or in *Pichia pastoris,* respectively, according to standard procedures. Both proteins were then purified by immobilized metal ion affinity chromatography, eluted from the column under denaturating conditions and stored in PBS containing 4M urea. The recombinant proteins were then intraperitoneally injected in Balb/c mice at a 1-week interval (on days 0, 7 and 14) and blood samplings were performed on days 0, 21, 28 and 35.

In mice, 3 injections of 200 mcg of IL31 Chimera A led to a sustained IgG production (Figure 3A). At the same dose, 3 injections of canonical IL31 elicited IgG production only in presence of alum (2 mg per injection). That IgG production was lower than those obtained with the Chimera. In addition, IgG production was not observed when canonical IL31 was mixed and not coupled to 20 mcg of a free TI peptide #60 having a sequence of SEQ ID NO: 21. In the same time, neither IgM, nor IgE were detected. Therefore, the mere addition of a TI segment to the carboxy-terminus of a non-immunogenic protein is sufficient to induce the production of IgG after injection into mice without addition of any adjuvant.

In order to determine whether IgG generated in response to Chimera can neutralize IL31 function, the inventors intravenously injected 10 mcg of natural IL31 protein on Day 71 post immunization and counted the number of scratching sequences during 35 min. IgG produced after injections of the Chimera in contrast to those produced after injection of the canonical IL31 are efficient to inhibit scratching induced by a subsequent administration of canonical IL31 (Figure 3B). Notably, this approach has been shown to prompt an immune response against a self-protein, i.e. to be able to break the immune tolerance and to block the activity of a potent cytokine.

### EXAMPLE 4: TI-assisted immunization against IL31 protein and kinetic of induction of antibodies reactive with canonical or TI segment of Chimera

Chimeric proteins have been generated by coupling various TI segments having a sequence of SEQ ID NO: 20, 21, 22 or 26 (of the section entitled "Sequences") to the IL31 protein. More particularly, four IL31 Chimera have been generated by coupling four different TI segments to the carboxy-terminal end of IL31 protein (IL31 Chimera A-D), produced in *E. coli* and purified as explained in Example 3. After 3 i.p. injections to mice (200 mcg each at a 1-week interval), IgG, but neither IgM nor IgE, have been produced in response to Chimera injections. The level of the IgG response varies depending on the TI segment coupled to IL31 Chimera (Figure 4A). The inventors have also observed that IgG produced after injections of the Chimera are efficient to inhibit scratching induced by a subsequent administration of canonical IL31 (Figure 4B). The results obtained show that the method of the invention has allowed to elicit neutralizing antibodies, namely IgG, and that some TI segments (A, D) are more efficient than others (B, C) to block mouse IL31-induced scratching in mice.

Then, the inventors have tested the impact of repeated IL31 Chimera injections (1, 2 or 3 injections of 200 mcg each at a 1-week interval) on IgG production and scratching reduction. Repeated injections were required to reach high titers of anti-IL31 IgG. Moreover, the titers of IL31 IgG remained elevated for at least 7 months post immunization (Figure 5A). One month after that time, these IgG remained efficient to significantly reduce spontaneous scratching observed in mice as well as IL31-induced scratching (Figure 5B). The results demonstrate that persistent IgG retained their neutralizing effect in the long term. Then two Chimera (A and D) have been generated by coupling TI segments to either the N-terminus or the C-terminus of IL31 protein, produced in *E. coli* and purified as explained in Example 3. These two Chimera have been injected in mice (2 to 5 injections of 40 mcg each at a 1-week interval) and the levels of IgG directed against either the canonical part, or the TI segment of the Chimera have been measured. Irrespective of the NH2- or COOH-terminal-position of the TI segment on the Chimera, high levels of IgG were detected in immunized mice (Figure 6). The ratio of antibodies reactive with canonical versus TI segments of the target decreased with increasing number of injections.

The inventors have thus demonstrated that injections of the chimeric scratching proteins IL31 according to the invention, induced the production of specific IgG in a very significant manner, thus causing inhibition of the cytokine-induced scratching. Noteworthy, IL31 is a potent cytokine active at the nanomolar range highlighting that the antibodies produced in response to IL31-TI Chimera display a particularly high affinity to counteract IL31 activity.

### EXAMPLE 5: TI-assisted immunization against a self-protein

Insulin is a hormone implicated in the regulation of glycemia. Only under pathologic conditions, antibodies directed against this self-protein are detectable in blood. The inventors designed and produced a construct, as explained in Example 3, derived to canonical insulin but modified to be inactive when injected to mice. Then, chimeric proteins (having a sequence of SEQ ID NO: 9, 59, 10 and 60) have been generated by coupling various TI segments (for example TI segments having a sequence of SEQ ID NO: 25 or 27) to the canonical insulin of SEQ ID NO: 8 or 58 (as enumerated in the section entitled "Sequences"). As observed above, 3 intraperitoneal injections (on Days 0, 14, and 28) of 130 mcg of insulin Chimera induced IgG against canonical insulin, but neither IgE, nor IgM (Figure 7A). The magnitude of this induction was dependent on the coupled TI segment (INS Chimera E > F) and significantly higher than with canonical insulin.

To determine the impact of the induction of anti-insulin IgG, glycemia was measured every 3-4 days by mandibular bleeding. One week after the last injection, glycemia was significantly increased in mice, where the level of anti-insulin IgG was the highest (i.e. in mice injected with INS Chimera E) (Figure 7B). Hence, the invention can be used to direct the synthesis of neutralizing lg against a self-protein. The invention can also be used to produce novel animal models of autoimmune disorders and dissect at the cellular level the interplay between humoral and cellular immunity.

### EXAMPLE 6: TI-assisted immunization against a non-self protein

IgE are antibodies associated with allergic conditions (e.g. rhinitis, asthma, atopic dermatitis, anaphylaxis...). Omalizumab (Xolair^{R}) is a monoclonal antibody targeting IgE and used to control severe and persistent allergic asthma and to treat chronic spontaneous urticarial. The inventors next tested whether a TI segment could also induced IgG when associated with a non-self-protein. Dog IgE has been chosen as a model and more precisely, the CH3 domain of dog IgE, because of its isotype and species specificities. Using 3-D structural prediction of the dog IgE, the inventors identified that the IgE-CH3 domain is highly solvent accessible (Figure 8A). Three injections, at a 1-week interval, of 100 mcg of a Chimera containing a canonical segment of dog IgE-CH3 Fc domain fused to the TI segment used in the previous example, elicited the production of IgG specifically reactive with dog IgE, without detectable cross reactivity with dog IgG (Figure 8B). Hence, TI segments were able to induce IgG production irrespective of the nature of the protein. The results show that the level of IgG increases very significantly after injection of IgE Chimera according to the invention, having for example a sequence of SEQ ID NO: 12 or 62 (as listed in the section "Sequences"). This would mimic the activity of a monoclonal antibody targeting IgE.

### EXAMPLE 7: TI-assisted immunization against SARS-CoV-2

SARS-CoV-2 coronavirus, was reported to be the causative agent of Covid-19, by Chinese authorities in December 2019 and WHO declared the pandemic on March 11, 2020. Spike protein or S protein lies on the outer envelope of the virus and leads to the entry of the virus into the human cells through interactions with Angiotensin Converting Enzyme-2 (ACE2).

Transcription infidelity and ribosomal frameshifts have been abundantly described as contributors to viral protein diversity (Atkins, 2016). We first aligned viral mRNA sequences to the reference genomic RNA to identify RNA-RNA bases Divergences (RRD) using bioinformatics procedures applying the stringent filters previously described (Brulliard, 2007; Thouvenot, 2020). In SARS-CoV-2, 891 gapped positions were identified (Figure 9A). Interestingly, the number of single base gap events in HCoV-HKU1, a benign human coronavirus, was 687. In most eukaryotic cells, RNAs gapped at a given position rarely exceed 1% of total mRNA (Thouvenot, 2020), but in SARS-CoV-2 frameshifted RNA represented as much as 15% of the total mRNA, while does not exceed 5% in HCoV-HKU1. In both viruses, these TI gaps are distributed on the entire viral genome and also affect the entire S transcript but strikingly, the S transcripts of SARS-CoV-2 contain far more TI events than that of HCov-HKU1 (p = 3.6e⁻¹⁵) (Figure 9B). In addition, *in silico* translation of gapped RNA revealed that predicted TI segments of SARS-CoV-2 TI proteins have higher isoelectric point, higher global charge and lower hydrophobicity than those from HCoV-HKU1. Hence, this higher gap density in the Spike protein transcripts likely leads to the translation to many Spike-TI proteins with carboxyterminal TI segments.

Natural Spike protein exhibits conformational plasticity that modulates the accessibility of the ACE2 binding motif (or RBD for Receptor Binding Domain) (Walls, 2019; Cai, 2020). These structural rearrangements may hinder antibody interaction, inventors therefore produced a construct centered on the RBD of S protein that contains five noncontiguous domains (Figure 10A; green, blue, red, grey and yellow). All domains are derived from the canonical S protein and all five are Exposed on the S (5ES) surface irrespective of the open or closed conformation of the trimer (Figure 10B). Several TI Chimera of that construct were generated by fusion of TI segments described above at 5ES carboxy-terminal end to produce 5ES-TI Chimera A and D having a sequence of SEQ ID NO: 14, 64, 15, 65, 16, 66, 17 or 67 (as listed in the section "Sequences").

Mice were first immunized once a day during eight days with 20 mcg of canonical 5ES and/or 5ES-TI Chimera, provided alone and in combination. All immunizations yielded, as soon as on Day 14, to an increase in IgG reactive with 5ES (Figure 11A). The 5ES IgG detected on Day 14 and Day 31 did not cross-react with the Full-Length S protein (FLS) (Figure 11B). Mice were maintained in their normal environment without further immunization until Day 36. On that day, they received a single injection of the same immunogens, which resulted, after 7 days (Day 43), in a significant increase in 5ES-specific IgG (Figure 11A). These IgG became cross-reactive with the FLS, especially when 5ES Chimera A and 5ES Chimera D were combined (Figure 11B). The capability of serum to block the interaction between S protein and ACE2 has been evaluated *in vitro* (SARS-CoV-2 Surrogate Virus Neutralizing Test kit, Genscript Biotech; Tan, 2020). As shown in Figure 11C, the combination of 5ES-TI Chimera A and D was more effective in the induction of serum neutralizing activity (SNA) than 5ES Chimera individually administrated. In mice sensitized, then boosted with the canonical 5ES construct alone, the SNA was not significantly increased (p= 0.78), while an increase in 5ES and FLS specific IgG was observed on Day 43 (p= 0.04). Furthermore, we verified that the boosts were well tolerated with none of the clinical signs typically observed in allergic/hyperimmunized mice. Hence, mouse immunizations with TI Chimera of 5ES but not with canonical 5ES construct induce SARS-CoV-2 neutralizing antibodies. Together, the data indicated that the presence, at the carboxy-terminal end of the immunogen, of a cationic segment translated from frameshifted RNA is required for the conversion of these 5ES IgG into neutralizing antibodies.

The combination of the 5ES-TI Chimera A and D yielded the highest neutralizing effect. We next ascertained the timeframe of the induction of the 5ES and FLS IgG reactivity and SNA (Figure 12A, B, C). Between Day 0 and Day 4 post-boost, the median growth of 5ES and FLS IgG reactivities were 69 % and 86 %, while, between Day 4 and Day 7, the median growth rates were 63 % and 179 %, respectively. This reflected the progressive acquisition of cross-reactivity of 5ES-IgG with the trimeric FLS. We found that a significant SNA increase was detectable as early as 4 days post-boost (Figure 12C). The median SNA grew by 57 % between Day 0 and Day 4, and by 77 % between Day 4 and Day 7.

In order to investigate the opportunity to treat Covid-19 patients by developing passive serotherapy using TI-assisted immunization, the inventors tested these findings in cows. The cow was selected because coronaviruses represent an important cause of calves' mortality by severe diarrhea and because cow may provide an abundant source of potentially neutralizing antibodies. A combination of the 5ES-TI Chimera A, B, C and D was intraperitoneally injected to cows once a day during eight days at two different doses (0.1 mg or 0.025 mg each). The induction of IgG to the canonical 5ES construct was detectable in all cows on Day 14 (and as early as Day 9 for Cow 22) (Figure 13A). A similar induction was obtained when the combination of the same four proteins (0.2 mg each per injection) was injected three times at a 1-week interval (Figure 13B).

### EXAMPLE 8: TI-assisted immunization against Staphylococcus aureus

*Staphylococcus aureus,* an opportunistic human pathogen, is the most common etiological agent of skin and soft tissue infections and may cause severe other diseases like endocarditis, nosocomial pneumonia, and septicemia. S. *aureus* encodes manganese ion transporters, playing an important role for its virulence. Among them, MntC is considered to be a good target for vaccine strategy because its presence on the cell surface, its early expression during the course of infection, and its high immunogenicity, eliciting a strong antibody response. Using prediction of solvent accessibility, the inventors identified three highly accessible regions located on the surface of MntC as putative epitopes (Figure 14A, red, green and purple boxes). A canonical *Staph* protein has been designed by the inventors joining these three sequences and *Staph* Chimera are generated by coupling any previously identified TI segment to the canonical protein comprising or consisting of SEQ ID NO: 68 or 18 (see also Figure 14B). Inventors assume that after production in *E. coli* or *Pichia pastoris* and purification, the Chimera injected to mice is able to induce neutralizing antibody production directed to *Staphyloccocus aureus.*

### EXAMPLE 9: TI-assisted immunization against Cryptosporidium

*Cryptosporidium parvum* is a parasitic protozoan causing infectious diarrhea in human and other animals. *C parvum* produces a gp60 glycoprotein that is proteolytically cleaved into two surface glycoproteins, gp40 and gp15. Two gp60 AA sequences have been reported in reference protein databases, both containing a predicted transmembrane (TM) domain at the carboxy-terminal end of the protein (Figure 15A). Gp15 is further targeted by the inventors because it is known to be key in mediating infection of host cells. Both Gp15 sequences are similar but in order to take sequence variation into account, canonical C1 and C2 sequences were retained. Hence, a canonical CRYPI protein containing both regions separated by two glycine residues has been designed by the inventors and any previously identified TI segment may be coupled to the canonical protein (comprising or consisting of SEQ ID NO: 69 or 19) to create CRYPI Chimera used for mouse immunization (Figure 15B).

### References

Atkins JF, Loughran G, Bhatt PR, Firth AE, Baranov PV. Ribosomal frameshifting and transcriptional slippage: From genetic steganography and cryptography to adventitious use. Nucleic Acids Res., 2016; 44, 7007-7078.
Balakrishnan T, Bela-Ong DB, Toh YX, Flamand M, Devi S, et al. Dengue Virus Activates Polyreactive, Natural IgG B Cells after Primary and Secondary Infection. PLoS ONE, 2011; 6(12): e29430.
Brulliard M, Lorphelin D, Collignon O, Lorphelin W, Thouvenot B, Gothie E, Jacquenet S, Ogier V, Roitel O, Monnez JM, Vallois P, Yen FT, Poch O, Guenneugues M, Karcher G, Oudet P, Bihain BE. Nonrandom variations in human cancer ESTs indicate that mRNA heterogeneity increases during carcinogenesis. Proc Natl Acad Sci, 2007; 104: 7522-7527.
Cai Y, Zhang J, Xiao T, Peng H, Sterling SM, Walsh RM, Rawson S, Rits-Volloch S, Chen B. Distinct conformational states of SARS-CoV-2 spike protein. Science, 2020; 369: 1586-1592.
Jordan D. Dimitrov, Cyril Planchais, Lubka T. Roumenina, Tchavdar L. Vassilev, Srinivas V. Kaveri and Sebastien Lacroix-Desmazes, Antibody Polyreactivity in Health and Disease: Statu Variabilis. J Immunol, 2013; 191 (3): 993-999.
Ju YS, Kim JI, Kim S, Hong D, Park H, Shin JY, Lee S, Lee WC, Kim S, Yu SB, Park SS, Seo SH, Yun JY, Kim HJ, Lee DS, Yavartanoo M, Kang HP, Gokcumen O, Govindaraju DR, Jung JH, Chong H, Yang KS, Kim H, Lee C, Seo JS. Extensive genomic and transcriptional diversity identified through massively parallel DNA and RNA sequencing of eighteen Korean individuals. Nat Genet, 2011; 43(8): 745-752.
Li M, Wang IX, Li Y, Bruzel A, Richards A, Toung JM, Cheung V. Widespread RNA and DNA Sequence Differences in the Human Transcriptome. Science, 2011; 333 (6038): 53-58.
Silverberg J, Pinter A, Pulka G, Poulin Y, Bouaziz JD, Wollenberg A, Murrell DF, Alexis A, Lindsey L, Ahmad F, Piketty C, Clucas A. Phase 2B randomized study of nemolizumab in adults with moderate-to-severe atopic dermatitis and severe pruritus. J Allergy Clin Immunol, 2020; 145: 173-182.
Song W, Gui M, Wang X, Xiang Y. Cryo-EM Structure of the SARS Coronavirus Spike Glycoprotein in Complex with Its Host Cell Receptor ACE2. PLOS Pathogens, 2018; 14 (8): e1007236.
Tan CW, Chia WN, Qin X, Liu P, Chen MIC, Tiu C, Hu Z, Chen VCW, Young BE, Sia WR, Tan YJ, Foo R, Yi Y, Lye DC, Anderson DE, Wang LF. A SARS-CoV-2 surrogate virus neutralization test based on antibody-mediated blockage of ACE2-spike protein-protein interaction. Nat Biotechnol, 2020; 38: 1073-1078.
Thouvenot B, Roitel O, Tomasina J, Hilselberger B, Richard C, Jacquenet S, Codreanu-Morel F, Morisset M, Kanny G, Beaudouin E, Delebarre-Sauvage C, Olivry T, Favrot C, Bihain BE. Transcriptional frameshifts contribute to protein allergenicity. J Clin Invest, 2020; 130(10): 5477-5492.
Walls AC, Xiong X, Park YJ, Tortorici MA, Snijder J, Quispe J, Cameroni E, Gopal R, Dai M, Lanzavecchia A, Zambon M, Rey FA, Corti D, Veesler D. Unexpected Receptor Functional Mimicry Elucidates Activation of Coronavirus Fusion. Cell, 2019; 176: 1026-1039.e15.
Wons E, Furmanek-Blaszk B, Sektas M. RNA Editing by T7 RNA Polymerase Bypasses InDel Mutations Causing Unexpected Phenotypic Changes. Nucleic Acids Research, 2015; 43: 3950-3963.

### Sequences

**Canonical proteins and Chimera** (all are recombinant proteins, either non tagged, or tagged (with His tag which is in bold), and wherein the TI segment is underlined)
Canonical IL31 (SEQ ID NO: 1) 
MKHHHHHHMSHMAPTHQLPPSDVRKIILELQPLSRGLLEDYQKKETGVPESNRTLLLCLTSDSQPPRLNSSAI
LPYFRAIRPLSDKNIIDKIIEQLDKLKFQHEPETEISVPADTFECKSFILTILQQFSACLESVFKSLNSGPQ
Canonical IL31 (SEQ ID NO: 70) 
MSHMAPTHQLPPSDVRKIILELQPLSRGLLEDYQKKETGVPESNRTLLLCLTSDSQPPRLNSSAILPYFRAIRPLS
DKNIIDKIIEQLDKLKFQHEPETEISVPADTFECKSFILTILQQFSACLESVFKSLNSGPQ
IL31 Chimera A (SEQ ID NO: 2) 
MKHHHHHHMSHMAPTHQLPPSDVRKIILELQPLSRGLLEDYQKKETGVPESNRTLLLCLTSDSQPPRLNSSAI
LPYFRAIRPLSDKNIIDKIIEQLDKLKFQHEPETEISVPADTFECKSFILTILQQFSACLESVFKSLNSGPQLKIQKLQ
GKCSQPGMVGCSLTTWRTSMVLGRNI
IL31 Chimera A (SEQ ID NO: 52) 
MSHMAPTHQLPPSDVRKIILELQPLSRGLLEDYQKKETGVPESNRTLLLCLTSDSQPPRLNSSAILPYFRAIRPLS
DKNIIDKIIEQLDKLKFQHEPETEISVPADTFECKSFILTILQQFSACLESVFKSLNSGPQLKIQKLQGKCSQPGMV
GCSLTTWRTSMVLGRNI
IL31 Chimera B (SEQ ID NO: 3) 
MKHHHHHHMSHMAPTHQLPPSDVRKIILELQPLSRGLLEDYQKKETGVPESNRTLLLCLTSDSQPPRLNSSAI
LPYFRAIRPLSDKNIIDKIIEQLDKLKFQHEPETEISVPADTFECKSFILTILQQFSACLESVFKSLNSGPQLQICAKY
SSVTAWTPAAGRSCKMEGCRWWR
IL31 Chimera B (SEQ ID NO: 53) 
MSHMAPTHQLPPSDVRKIILELQPLSRGLLEDYQKKETGVPESNRTLLLCLTSDSQPPRLNSSAILPYFRAIRPLS
DKNIIDKIIEQLDKLKFQHEPETEISVPADTFECKSFILTILQQFSACLESVFKSLNSGPQLQICAKYSSVTAWTPA
AGRSCKMEGCRWWR
IL31 Chimera C (SEQ ID NO: 4) 
MKHHHHHHMSHMAPTHQLPPSDVRKIILELQPLSRGLLEDYQKKETGVPESNRTLLLCLTSDSQPPRLNSSAI
LPYFRAIRPLSDKNIIDKIIEQLDKLKFQHEPETEISVPADTFECKSFILTILQQFSACLESVFKSLNSGPQLCFKRYE
LRLPAKRKRKRN
IL31 Chimera C (SEQ ID NO: 54) 
MSHMAPTHQLPPSDVRKIILELQPLSRGLLEDYQKKETGVPESNRTLLLCLTSDSQPPRLNSSAILPYFRAIRPLS
DKNIIDKIIEQLDKLKFQHEPETEISVPADTFECKSFILTILQQFSACLESVFKSLNSGPQLCFKRYELRLPAKRKRK
RN
IL31 Chimera D (SEQ ID NO: 5) 
MKHHHHHHMSHMAPTHQLPPSDVRKIILELQPLSRGLLEDYQKKETGVPESNRTLLLCLTSDSQPPRLNSSAI
LPYFRAIRPLSDKNIIDKIIEQLDKLKFQHEPETEISVPADTFECKSFILTILQQFSACLESVFKSLNSGPQVKSPQK
SYLFPSSMIGIGSLPSCWACWIQQ
IL31 Chimera D (SEQ ID NO: 55) 
MSHMAPTHQLPPSDVRKIILELQPLSRGLLEDYQKKETGVPESNRTLLLCLTSDSQPPRLNSSAILPYFRAIRPLS
DKNIIDKIIEQLDKLKFQHEPETEISVPADTFECKSFILTILQQFSACLESVFKSLNSGPQVKSPQKSYLFPSSMIGI
GSLPSCWACWIQQ
IL31 Chimera with TI segment at COOH-terminus (SEQ ID NO: 6) 
MKHHHHHHMTCSLSFGAPISKEDLRTTIDLLKQESQDLYNNYSIKQASGMSADESIQLPCFSLDREALTNISVI
IAHLEKVKVLSENTVDTSWVIRWLTNISCFNPLNLNISVPGNTDESYDCKVFVLTVLKQFSNCMAELQAKDNT
TCLKIQKLQGKCSQPGMVGCSLTTWRTSMVLGRNI
IL31 Chimera with TI segment at COOH-terminus (SEQ ID NO: 56) 
MTCSLSFGAPISKEDLRTTIDLLKQESQDLYNNYSIKQASGMSADESIQLPCFSLDREALTNISVIIAHLEKVKVLS
ENTVDTSWVIRWLTNISCFNPLNLNISVPGNTDESYDCKVFVLTVLKQFSNCMAELQAKDNTTCLKIQKLQG
KCSQPGMVGCSLTTWRTSMVLGRNI
IL31 Chimera with TI segment at NH2-terminus (SEQ ID NO: 7) 
MKHHHHHHMLKIQKLQGKCSQPGMVGCSLTTWRTSMVLGRNIMTCSLSFGAPISKEDLRTTIDLLKQESQ
DLYNNYSIKQASGMSADESIQLPCFSLDREALTNISVIIAHLEKVKVLSENTVDTSWVIRWLTNISCFNPLNLNIS
VPGNTDESYDCKVFVLTVLKQFSNCMAELQAKDNTTC
IL31 Chimera with TI segment at NH2-terminus (SEQ ID NO: 57) 
LKIQKLQGKCSQPGMVGCSLTTWRTSMVLGRNIMTCSLSFGAPISKEDLRTTIDLLKQESQDLYNNYSIKQAS
GMSADESIQLPCFSLDREALTNISVIIAHLEKVKVLSENTVDTSWVIRWLTNISCFNPLNLNISVPGNTDESYDC
KVFVLTVLKQFSNCMAELQAKDNTTC
INS=insulin
Canonical INS (SEQ ID NO: 8) 
MKHHHHHHMFVKQHLCGPHLVEALYLVCGERGFFYTPKSGGGFVKQHLCGSHLVEALYLVCGERGFFYTP
MSGGGIVDQCCTSICSLYQLENYCN
Canonical INS (SEQ ID NO: 58) 
MFVKQHLCGPHLVEALYLVCGERGFFYTPKSGGGFVKQHLCGSHLVEALYLVCGERGFFYTPMSGGGIVDQ
CCTSICSLYQLENYCN
INS Chimera E (SEQ ID NO: 9) 
MKHHHHHHMFVKQHLCGPHLVEALYLVCGERGFFYTPKSGGGFVKQHLCGSHLVEALYLVCGERGFFYTPM
SGGGIVDQCCTSICSLYQLENYCNFLISMPEKCVNTWIMKI
INS Chimera E (SEQ ID NO: 59) 
MFVKQHLCGPHLVEALYLVCGERGFFYTPKSGGGFVKQHLCGSHLVEALYLVCGERGFFYTPMSGGGIVDQC
CTSICSLYQLENYCNFLISMPEKCVNTWIMKI
INS Chimera F (SEQ ID NO: 10) 
MKHHHHHHMFVKQHLCGPHLVEALYLVCGERGFFYTPKSGGGFVKQHLCGSHLVEALYLVCGERGFFYTPM
SGGGIVDQCCTSICSLYQLENYCNEFDIRRIRQMLIITK
INS Chimera F (SEQ ID NO: 60) 
MFVKQHLCGPHLVEALYLVCGERGFFYTPKSGGGFVKQHLCGSHLVEALYLVCGERGFFYTPMSGGGIVDQC
CTSICSLYQLENYCNEFDIRRIRQMLIITK
Dog IgE-CH3 (SEQ ID NO: 11) 
MKHHHHHHMFIPPTVKLFHSSCNPVGDTHTTIQLLCLISGYVPGDMEVIWLVDGQKATNIFPYTAPGTKEG
NVTSTHSELNITQGEWVSQKTY
Dog IgE-CH3 (SEQ ID NO: 61) 
MFIPPTVKLFHSSCNPVGDTHTTIQLLCLISGYVPGDMEVIWLVDGQKATNIFPYTAPGTKEGNVTSTHSELNI
TQGEWVSQKTY
Dog IgE-CH3 Chimera A (SEQ ID NO: 12) 
MKHHHHHHMFIPPTVKLFHSSCNPVGDTHTTIQLLCLISGYVPGDMEVIWLVDGQKATNIFPYTAPGTKEG
NVTSTHSELNITQGEWVSQKTYLKIQKLQGKCSQPGMVGCSLTTWRTSMVLGRNI
Dog IgE-CH3 Chimera A (SEQ ID NO: 62) 
MFIPPTVKLFHSSCNPVGDTHTTIQLLCLISGYVPGDMEVIWLVDGQKATNIFPYTAPGTKEGNVTSTHSELNI

TQGEWVSQKTYLKIQKLQGKCSQPGMVGCSLTTWRTSMVLGRNI
Canonical 5ES (for SARS-CoV-2 coronavirus) (SEQ ID NO: 13) 
MKHHHHHHMVYYHKNNKSWMESEFRVYSSANNCTFEYVSQPFLMDLEGKQGNFKNGGKRISNCVADYS
VLYNSASFSTFKCYGVSPTKLNDLCGGSTEIYQAGSTPCNGVEGFNCYFPLQSYGFQPTGGVCGPKKSTNLV
KNKCVNFNFGGDKNTQEVFAQVKQIYKTPPIKDFGG
Canonical 5ES (for SARS-CoV-2 coronavirus) (SEQ ID NO: 63) 
MVYYHKNNKSWMESEFRVYSSANNCTFEYVSQPFLMDLEGKQGNFKNGGKRISNCVADYSVLYNSASFST
FKCYGVSPTKLNDLCGGSTEIYQAGSTPCNGVEGFNCYFPLQSYGFQPTGGVCGPKKSTNLVKNKCVNFNF
GGDKNTQEVFAQVKQIYKTPPIKDFGG
5ES-TI Chimera A (for SARS-CoV-2 coronavirus) (SEQ ID NO: 14) 
MKHHHHHHMVYYHKNNKSWMESEFRVYSSANNCTFEYVSQPFLMDLEGKQGNFKNGGKRISNCVADYS
VLYNSASFSTFKCYGVSPTKLNDLCGGSTEIYQAGSTPCNGVEGFNCYFPLQSYGFQPTGGVCGPKKSTNLV
KNKCVNFNFGGDKNTQEVFAQVKQIYKTPPIKDFGGPSGPSWASWLRCWCWS
5ES-TI Chimera A (for SARS-CoV-2 coronavirus) (SEQ ID NO: 64) 
MVYYHKNNKSWMESEFRVYSSANNCTFEYVSQPFLMDLEGKQGNFKNGGKRISNCVADYSVLYNSASFST
FKCYGVSPTKLNDLCGGSTEIYQAGSTPCNGVEGFNCYFPLQSYGFQPTGGVCGPKKSTNLVKNKCVNFNF
GGDKNTQEVFAQVKQIYKTPPIKDFGGPSGPSWASWLRCWCWS
5ES-TI Chimera B (for SARS-CoV-2 coronavirus) (SEQ ID NO: 15) 
MKHHHHHHMVYYHKNNKSWMESEFRVYSSANNCTFEYVSQPFLMDLEGKQGNFKNGGKRISNCVADYS
VLYNSASFSTFKCYGVSPTKLNDLCGGSTEIYQAGSTPCNGVEGFNCYFPLQSYGFQPTGGVCGPKKSTNLV
KNKCVNFNFGGDKNTQEVFAQVKQIYKTPPIKDFGGVKSPQKSYLFPSSMIGIGSLPSCWACWIQQ
5ES-TI Chimera B (for SARS-CoV-2 coronavirus) (SEQ ID NO: 65) 
MVYYHKNNKSWMESEFRVYSSANNCTFEYVSQPFLMDLEGKQGNFKNGGKRISNCVADYSVLYNSASFST
FKCYGVSPTKLNDLCGGSTEIYQAGSTPCNGVEGFNCYFPLQSYGFQPTGGVCGPKKSTNLVKNKCVNFNF
GGDKNTQEVFAQVKQIYKTPPIKDFGGVKSPQKSYLFPSSMIGIGSLPSCWACWIQQ
5ES-TI Chimera C (for SARS-CoV-2 coronavirus) (SEQ ID NO: 16) 
MKHHHHHHMVYYHKNNKSWMESEFRVYSSANNCTFEYVSQPFLMDLEGKQGNFKNGGKRISNCVADYS
VLYNSASFSTFKCYGVSPTKLNDLCGGSTEIYQAGSTPCNGVEGFNCYFPLQSYGFQPTGGVCGPKKSTNLV
KNKCVNFNFGGDKNTQEVFAQVKQIYKTPPIKDFGGLKIQKLQGKCSQPGMVGCSLTTWRTSMVLGRNI
5ES-TI Chimera C (for SARS-CoV-2 coronavirus) (SEQ ID NO: 66) 
MVYYHKNNKSWMESEFRVYSSANNCTFEYVSQPFLMDLEGKQGNFKNGGKRISNCVADYSVLYNSASFST
FKCYGVSPTKLNDLCGGSTEIYQAGSTPCNGVEGFNCYFPLQSYGFQPTGGVCGPKKSTNLVKNKCVNFNF
GGDKNTQEVFAQVKQIYKTPPIKDFGGLKIQKLQGKCSQPGMVGCSLTTWRTSMVLGRNI
5ES-TI Chimera D (for SARS-CoV-2 coronavirus) (SEQ ID NO: 17) 
MKHHHHHHMVYYHKNNKSWMESEFRVYSSANNCTFEYVSQPFLMDLEGKQGNFKNGGKRISNCVADYS
VLYNSASFSTFKCYGVSPTKLNDLCGGSTEIYQAGSTPCNGVEGFNCYFPLQSYGFQPTGGVCGPKKSTNLV
KNKCVNFNFGGDKNTQEVFAQVKQIYKTPPIKDFGGSAKLSWTTFQILSWKPMFHFWY
5ES-TI Chimera D (for SARS-CoV-2 coronavirus) (SEQ ID NO: 67) 
MVYYHKNNKSWMESEFRVYSSANNCTFEYVSQPFLMDLEGKQGNFKNGGKRISNCVADYSVLYNSASFST
FKCYGVSPTKLNDLCGGSTEIYQAGSTPCNGVEGFNCYFPLQSYGFQPTGGVCGPKKSTNLVKNKCVNFNF
GGDKNTQEVFAQVKQIYKTPPIKDFGGSAKLSWTTFQILSWKPMFHFWY
Canonical *Staph* protein (for *Staphylococcus aureus*) (SEQ ID NO: 18) 
MKHHHHHHQLEKLNNDSKDKFNDIPKEQRAMITSEGAFKYFSKQYGITPGYIWEINTEKQGTPEQMRQAIE
FVKKHKLKHLLVETSVDKKAMESLSEETKKDIFGE
Canonical Staph protein (for Staphylococcus aureus) (SEQ ID NO: 68) 
QLEKLNNDSKDKFNDIPKEQRAMITSEGAFKYFSKQYGITPGYIWEINTEKQGTPEQMRQAIEFVKKHKLKHL

LVETSVDKKAMESLSEETKKDIFGE
Canonical CRYPI protein (for *Cryptosporidium parvum)* (SEQ ID NO: 19) 
MKHHHHHHSLTEETSETATVDLFAFTLNGGKRIEVAVPNAEETSKRDKYSLVADDSAFYTGKNSGSTDGVYKL
NENGDLVDKDNKVLLKDAGSSGGSLSEETSEAAATVDLFAFTLDGGKRIEVAVPNVEDASKRDKYSLVADDKP
FYTGANSGTTNGVYRLNENGDLVDKDNTVLLKDAGSS
Canonical CRYPI protein (for *Cryptosporidium parvum)* (SEQ ID NO: 69)

SLTEETSETATVDLFAFTLNGGKRIEVAVPNAEETSKRDKYSLVADDSAFYTGKNSGSTDGVYKLNENGDLVDK
DNKVLLKDAGSSGGSLSEETSEAAATVDLFAFTLDGGKRIEVAVPNVEDASKRDKYSLVADDKPFYTGANSGT
TNGVYRLNENGDLVDKDNTVLLKDAGSS

### TI segments/peptides

- #7: LCFKRYELRLPAKRKRKRN (SEQ ID NO: 20)
- #60: LKIQKLQGKCSQPGMVGCSLTTWRTSMVLGRNI (SEQ ID NO: 21)
- #66: VKSPQKSYLFPSSMIGIGSLPSCWACWIQQ (SEQ ID NO: 22)
- #158: PSGPSWASWLRCWCWS (SEQ ID NO: 23)
- #162: SAKLSWTTFQILSWKPMFHFWY (SEQ ID NO: 24)
- #245: EFDIRRIRQMLIITK (SEQ ID NO: 25)
- #294: LQICAKYSSVTAWTPAAGRSCKMEGCRWWR (SEQ ID NO: 26)
- #367: FLISMPEKCVNTWIMKI (SEQ ID NO: 27)
- #1: WRRWAAPWFWSSLQLGGLVFRGPGPRARSR (SEQ ID NO: 28)
- #24: WTKWRLSWPWKTNLGLKFL (SEQ ID NO: 29)
- #25: RFPARAPWRCRAPRSTCCIPATIRWPRAST (SEQ ID NO: 30)
- #38: HLKCKLKKFDRWDPIKRGQ (SEQ ID NO: 31)
- #39: LLCCFSAFPSFLLKDGRRFSSPGWWSC (SEQ ID NO: 32)
- #44: WSIWLSREQRIGLAVPWRRRWRAWGPILMP (SEQ ID NO: 33)
- #76: MLWMVQKAWLEARKYWILVHQSKFLLVLRLWAES (SEQ ID NO: 34)
- #87: LQICGKCSSVTAWTLAAGRSCKMEGCRWWK (SEQ ID NO: 35)
- #90: WRKRKRNWPRKQGRRLLTIWLLGKMNELGS (SEQ ID NO: 36)
- #95: LCAPRRSLLSVIGQKNLRNSTAK (SEQ ID NO: 37)
- #92: LTAPRAWRARTASPWTAATVASGRRKTRLT (SEQ ID NO: 38)
- #131: IPKDRLQHQQKLQWLMPYMKYQ (SEQ ID NO: 39)
- #134: MRSTLSGRDFHPCRWLMKSLAF (SEQ ID NO: 40)
- #210: LGLGRNGIRNHRNPETLQLKFVVIGK (SEQ ID NO: 41)
- #221: TLIPRGTRKHCIWVLS (SEQ ID NO: 42)
- #234: KIQKLQGKCSQPGMV (SEQ ID NO: 43)
- #289: QRKPRRRPRERALSCTRTLQIRKPHPVANL (SEQ ID NO: 44)
- #290: RCFKRCALRLPAKRRRKRN (SEQ ID NO: 45)
- #291: TWPRTSCGCERNCRRRCSRERKPKAPCSHS (SEQ ID NO: 46)
- #292: WRKKKRNWLRRQGRRLLTTWPPERMSEHAS (SEQ ID NO: 47)
- #293: HLKCKLKKSGR (SEQ ID NO: 48)
- #295: MLWMALKAWLEARKYWIQGHQSKFLLVLRPWAES (SEQ ID NO: 49)
- #296: FAKLSWTTYQILSWRPMSLY (SEQ ID NO: 50)
- Canonical control peptide: ALLQKVRAEIASKEKEEEE (SEQ ID NO: 51)

## Claims

1. A chimeric immunogen comprising (i) one or more antigen(s) or fragment(s) thereof, covalently coupled to (ii) one or more TI segments.

2. The chimeric immunogen of claim 1, wherein the coupling is covalent, through a peptide bond, direct or through a linker group, either at C-terminal extremity or N-terminal extremity, or at both extremities of the TI segment(s).

3. The chimeric immunogen of claim 1 or 2, wherein the antigen is a protein.

4. The chimeric immunogen of any one of claims 1 to 3, wherein the antigen is a self-antigen.

5. The chimeric immunogen of any one of claims 1 to 3, wherein the antigen is a non-self-antigen, preferably a prion, viral, bacterial, parasite, mammalian, fish or avian protein.

6. The chimeric immunogen of claim 5, wherein the viral antigen is a coronavirus antigen such as a SARS-CoV-2 antigen.

7. The chimeric immunogen of claim 6, wherein the SARS-CoV-2 antigen is selected from Full Length Spike (FLS) protein and five most Exposed Spike domains (5ES).

8. The chimeric immunogen of any one of claims 1 to 7, wherein the TI segment comprises, consists essentially of, or consists of a sequence selected from any of SEQ ID NOs: 20 to 50.

9. A composition comprising a chimeric immunogen of any one of claims 1 to 8, and a pharmaceutically or veterinary acceptable excipient.

10. The composition of claim 9, which is a vaccine.

11. The chimeric immunogen of any one of claims 1 to 8, or the composition of claim 9 or 10, for use in the treatment of a viral disease, bacterial disease, parasite disease, auto-immune disease, prion disease, inflammatory disease, endometriosis, cancer, or a neurodegenerative disease.

12. The chimeric immunogen of any one of claims 1 to 8, or the composition of claim 9 or 10, for use in a method for inducing antigen-specific immunoglobulins in a subject, wherein the subject is human or non-human animal, preferably a mammal, e.g., a human, dog, cat, horse, cow or pig; an avian or a fish.

13. The chimeric immunogen or composition for use of claim 12, wherein the immunoglobulins are IgGs, IgMs, IgEs or IgAs, preferably IgGs or a mixture thereof.

14. The chimeric immunogen or composition for use of claim 13, wherein the IgGs have an affinity of 10E-9 or 10E-10 M or less.

15. The chimeric immunogen or composition for use of claim 12, wherein the method further comprises a step of collecting a serum sample from said subject, said serum sample comprising antigen-specific immunoglobulins.

16. The chimeric immunogen or composition for use of claim 15, wherein the serum sample comprises anti-viral immunoglobulins, preferably anti-coronaviral immunoglobulins such as anti-SARS-CoV-2 specific immunoglobulins.

17. The anti-SARS-CoV-2 specific immunoglobulins obtained from the serum sample of claim 15 or 16, for use in the treatment of Covid-19 by serotherapy.

18. The chimeric immunogen of any one of claims 1 to 8, or the composition of claim 9 or 10, for use in a method for prophylactically or therapeutically vaccinating a subject against a pathogen.

19. The chimeric immunogen or composition for use of claim 18, wherein the pathogen is SARS-CoV-2, and the antigen is selected from Full Length Spike (FLS) protein and five most Exposed Spike domains (5ES).

20. The chimeric immunogen or composition for use of claim 18 or 19, for reducing Covid-19 conversion to severity.

21. The chimeric immunogen of any one of claims 1 to 8, or the composition of claim 9 or 10, for use in a method for stimulating an immune response against an antigen, preferably a self-antigen, in a subject.

22. The chimeric immunogen of any one of claims 1 to 8, or the composition of claim 9 or 10, for use in a method for inducing an anti-IgE IgG immune response in a subject, wherein the method comprises administering to the subject at least one chimeric immunogen comprising an IgE, or a fragment thereof, coupled to one or more TI segments.

23. The chimeric immunogen of any one of claims 1 to 8, or the composition of claim 9 or 10, for use in a method for neutralizing IgE in a subject, wherein the method comprises administering to the subject at least one chimeric immunogen comprising an IgE, or a fragment thereof, coupled to one or more TI segments.

24. A method of increasing immunogenicity of a antigen, comprising coupling said antigen to a TI segment.

25. A method of producing a chimeric immunogen, comprising coupling at least one antigen to at least one TI segment.
